(19) **Europäisches Patentamt / European Patent Office / Office européen des brevets**

(11) **EP 3 231 362 A1**

(12) **EUROPEAN PATENT APPLICATION**
published in accordance with Art. 153(4) EPC

(43) Date of publication:
**18.10.2017 Bulletin 2017/42**

(51) Int Cl.:
**A61B 5/0245** (2006.01)

(21) Application number: **14907921.2**

(22) Date of filing: **12.12.2014**

(86) International application number:
**PCT/JP2014/083059**

(87) International publication number:
**WO 2016/092707 (16.06.2016 Gazette 2016/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Fujitsu Limited**
**Kawasaki-shi, Kanagawa 211-8588 (JP)**

(72) Inventors:
• **HOTTA, Shinji**
  **Kawasaki-shi**
  **Kanagawa 211-8588 (JP)**
• **INOMATA, Akihiro**
  **Kawasaki-shi**
  **Kanagawa 211-8588 (JP)**

(74) Representative: **Haseltine Lake LLP**
**Lincoln House, 5th Floor**
**300 High Holborn**
**London WC1V 7JH (GB)**

(54) **MEAL INTAKE ESTIMATION PROGRAM, MEAL INTAKE ESTIMATION METHOD, AND MEAL INTAKE ESTIMATION DEVICE**

(57)     A health care support system (1) acquires time-series data of heart rate; calculates a feature quantity relating to a second peak appearing after a first peak in which a peak of the heart rate appears first after start of a meal, for each partial data included in the time-series data of the heart rate; determines presence of a meal in the partial data using the feature quantity relating to the second peak calculated for each of the partial data; and estimates a meal time from the partial data determined to include a meal.

FIG.1

**Description**

[Technical Field]

The present invention relates to a meal estimation program, a meal estimation method, and a meal estimation apparatus.

[Background Art]

Health care has attracted attention. Health care includes prevention of lifestyle-related diseases, such as metabolic syndrome and diabetes, diets, and medical services. Performing such health care needs a process of recording lifestyle habits such as regular exercise and meals, noticing problems of one's lifestyle habits, and improving them.

For example, as prevention measures relating to "meal", a method for controlling meals is mentioned as follows, such as "when", "what", and "how much". Specifically, the method includes items of "having three meals regularly (when)", "having breakfast (when)", "taking nutrition with well balance (what)", "not taking too much calories (how much)", and "take less salt (what)".

For example, a record as to "when" the user had meals enables detection of irregular dietary habits, and execution of service of providing advice to prevent diseases.

For example, a meal detection system, a speech and eating state detection system, and a meal detection apparatus have been presented, as examples of a technique of performing determination as to diet. For example, the meal detection system detects a movement of raising and lowering the arm in eating food using an acceleration sensor, to determine the user's meal. The speech and eating state detection system uses mastication in eating food, and detects a frequency pattern specific to the sound of mastication inside the body. In the meal detection apparatus, under the situation in which an infrared sensor is installed on the table or the like, the human body is detected in the vicinity of the table, and thereafter threshold processing is performed as to whether the human body frequently moves.

However, in each of these techniques, because the way of having a meal is restricted to estimate the meal, or the place to estimate the meal is restrained, these techniques may lack versatility. For example, the trend of acceleration assumed in the meal detection system only corresponds to an aspect of the movement of the arm performed in eating food. When a movement of the arm other than the above is performed, because the movement has a different trend of acceleration, miss of detection occurs. In addition, in the speech and eating state detection system, because a microphone is attached to the user's neck at the time of having a meal, a burden is imposed on the user's body, and the user's external appearance deteriorates. The meal detection apparatus is capable of recognizing a meal only in a fixed environment, such as a place in which the infrared sensor is installed.

A life management terminal apparatus has also been presented, as an example of a technique using pulse waves for determining meal. The life management terminal apparatus determines that the user is taking a meal, when the pulse rate increases and no rapid increase exists in skin conductivity, in addition to appearance of mastication features that would occur when having a meal.

[Citation List]

[Patent Citation]

Patent Document

Patent Document 1: Japanese Laid-open Patent Publication No. 2011-115508
Patent Document 2: Japanese Laid-open Patent Publication No. 2012-61790
Patent Document 3: Japanese Laid-open Patent Publication No. 2004-81471
Patent Document 4: Japanese Laid-open Patent Publication No. 2010-158267
Patent Document 5: Japanese Laid-open Patent Publication No. 2007-48180
Patent Document 6: Japanese National Publication of International Patent Application No. 10-504739
Patent Document 7: Japanese Laid-open Patent Publication No. 2003-173375
Non Patent Document 1: Yuji MATSUDA, Hajime HOASHI, and Keiji YANAI, "RECOGNITION OF MULTIPLE-FOOD IMAGES BY DETECTING CANDIDATE REGIONS", Meeting on Image Recognition and Understanding (MIRU11), July 2011, http://img.cs.uec.ac.jp/pub/conf11/110720hoashi_0.pdf

[Summary of Invention]

[Technical Problem]

However, the techniques described above may cause erroneous determination in determination of meal.

Specifically, the life management terminal apparatus described above uses skin conductivity in determination of meal. Because the measurement accuracy of the skin conductivity decreases in sweating or the like, the possibility of erroneous determination also increases in determination of meal. In addition, when only the pulse rate is used without using the skin conductivity in the life management terminal apparatus described above, the pulse rate increases due to causes other than meal, such as mental strain, change in environmental temperature, and exercise action; erroneous determination may occur also in this case.

According to an aspect, an object of the present invention is to provide a meal estimation program, a meal estimation method, and a meal estimation apparatus capable of suppressing decrease in accuracy of determination of meal.

[Solution to Problem]

A meal estimation program according to one aspect causes a computer to execute a process including: acquiring time-series data of heart rate; calculating a feature quantity relating to a second peak appearing after a first peak in which a peak of the heart rate appears first after start of a meal, for each partial data included in the time-series data of the heart rate; determining presence of a meal in the partial data using the feature quantity relating to the second peak calculated for each of the partial data; and estimating a meal time from the partial data determined to include a meal.

[Advantageous Effects of Invention]

The present invention suppresses decrease in accuracy of determination of meal.

[Brief Description of Drawings]

FIG. 1 is a diagram illustrating a configuration of a health care support system according to a first embodiment.
FIG. 2 is a diagram illustrating an example of heart rate data.
FIG. 3 is a diagram illustrating an example of heart rate data.
FIG. 4 is a diagram illustrating an example of heart rate data.
FIG. 5 is a diagram illustrating an example of a method for calculating feature quantities (1).
FIG. 6 is a diagram illustrating an example of a method for calculating feature quantities (2).
FIG. 7 is a diagram illustrating an example of a method for calculating the feature quantities (2).
FIG. 8 is a diagram illustrating an example of a method for calculating the feature quantities (2).
FIG. 9 is a diagram illustrating an example of a method for calculating the feature quantities (2).
FIG. 10 is a diagram illustrating an example of a method for calculating feature quantities (3).
FIG. 11 is a diagram illustrating an example of a method for calculating feature quantities (4).
FIG. 12 is a diagram illustrating an example of a method for calculating the feature quantities (4).
FIG. 13 is a diagram illustrating an example of a method for calculating feature quantities (5).
FIG. 14 is a diagram illustrating an example of acceleration data.
FIG. 15 is a diagram illustrating an example of a method for removing noise heart rate.
FIG. 16 is a diagram illustrating an example of a method for calculating a removal period based on change points.
FIG. 17 is a diagram illustrating an example of a method for preparing window data.
FIG. 18 is a diagram illustrating an example of acceleration data.
FIG. 19 is a diagram illustrating an example of an identification boundary.
FIG. 20 is a diagram illustrating an example of health care support service.
FIG. 21 is a diagram illustrating an example of health care support service.
FIG. 22 is a diagram illustrating an example of heart rate data.
FIG. 23 is a diagram illustrating an example of acceleration data.
FIG. 24 is a diagram illustrating an example of a correlation table between the heart rate, the number of times of eating actions, and the exercise period.
FIG. 25 is a diagram illustrating an example of removing the noise heart rate.
FIG. 26 is a diagram illustrating an example of a result of extraction of window data.

FIG. 27 is a diagram illustrating an example of window data.

FIG. 28 is a diagram illustrating an example of a method for determining the pre-meal heart rate.

FIG. 29 is a diagram illustrating an example of a process of calculating the feature quantities.

FIG. 30 is a diagram illustrating an example of window data.

FIG. 31 is a diagram illustrating an example of the process of calculating the feature quantities.

FIG. 32 is a diagram illustrating an example of the process of calculating the feature quantities.

FIG. 33 is a diagram illustrating an example of the process of calculating the feature quantities.

FIG. 34 is a diagram illustrating an example of an approximation error.

FIG. 35 is a diagram illustrating an example of the process of calculating the feature quantities.

FIG. 36 is a diagram illustrating an example of an observation feature quantity list.

FIG. 37 is a diagram illustrating an example of an eating action number-of-times list.

FIG. 38 is a diagram illustrating an example of teacher data.

FIG. 39 is a diagram illustrating an example of a meal estimation model.

FIG. 40 is a diagram illustrating an example of a result of estimation of presence of meal.

FIG. 41 is a diagram illustrating an example of meal determination using the eating action number-of-times list.

FIG. 42 is a flowchart illustrating a process of the whole flow according to the first embodiment.

FIG. 43 is a flowchart illustrating a calculation processing process of the feature quantities (1).

FIG. 44 is a flowchart illustrating a calculation processing process of the feature quantities (2).

FIG. 45 is a flowchart illustrating a calculation processing process of the feature quantities (3).

FIG. 46 is a flowchart illustrating a calculation processing process of the feature quantities (4).

FIG. 47 is a flowchart illustrating a calculation processing process of the feature quantities (5).

FIG. 48 is a diagram illustrating an example of a hardware configuration of a computer executing the meal estimation program according to the first embodiment and a second embodiment.

[Embodiments for Carrying Out the Invention]

The following is explanation of a meal estimation program, a meal estimation method, and a meal estimation apparatus according to the present application with reference to attached drawings. The embodiments do not limit the disclosed technique. In addition, the embodiments may be properly combined within a scope not causing contradiction between the processing details.

First Embodiment

System Configuration

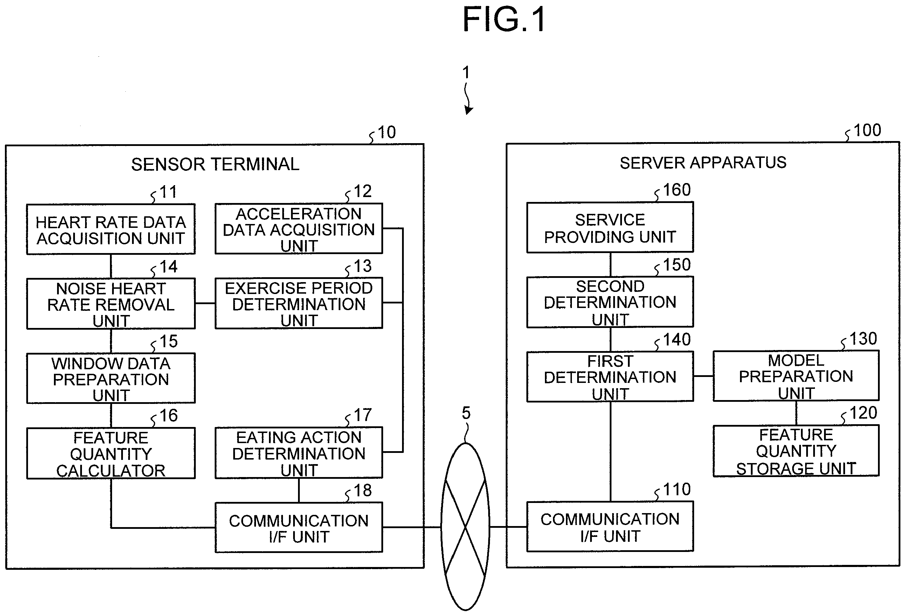

FIG. 1 is a diagram illustrating a configuration of a health care support system according to a first embodiment. The health care support system 1 illustrated in FIG. 1 provides various health care support services. For example, examples of the health care support services include a service of recording the life action, such as the meal time, of the user of a sensor terminal 10 using sensing data collected with the sensor terminal 10, and a derivative service using the record.

As part of the health care support services, the health care support system 1 uses feature quantities relating to two heart rate change peaks appearing after start of the meal, when the user's meal time is estimated from the time-series data of the heart rate. In this manner, the health care support system 1 suppresses estimation of the meal time at the scene in which the heart rate increases due to causes other than the meal, such as mental strain, change in environmental temperature, and exercise action, and suppresses erroneous determination of the meal time.

In the following explanation, the peak appearing first after the start of the meal among the two heart rate change peaks may be referred to as "first peak", and the peak appearing following the first peak may be referred to as "second peak".

As illustrated in FIG. 1, the health care support system 1 contains the sensor terminal 10, and a server apparatus 100. FIG. 1 illustrates the case where one sensor terminal 10 is contained; however, the health care support system 1 may contain a plurality of sensor terminals.

The sensor terminal 10 and the server apparatus 100 are connected through a network 5 to communicate with each other. The network 5 may be a communication network of a desired type, wired or wireless, such as a local area network including a local area network (LAN) and a virtual private network (VPN), and the Internet.

The sensor terminal 10 is a terminal device provided with a sensor.

As an embodiment, the sensor terminal 10 may be a terminal device dedicated for health care, a wearable gadget such as a smart glass and a smart watch, or a mobile terminal device. The category of the mobile terminal device includes a tablet terminal and a slate terminal, as well as a mobile communication terminal such as a smartphone, a mobile phone, and a personal handy phone system (PHS).

At least a heartbeat sensor is mounted on the sensor terminal 10. Using the heartbeat sensor, the sensor terminal 10 detects, for example, the heart rate per unit time of the user using the sensor terminal 10. The time-series data of the heart rate sensed using the heartbeat sensor as described above is used for calculating the feature quantities and estimating the meal start time. In the following explanation, the time-series data of the heart rate sensed using the heartbeat sensor may also be referred to as "heart rate data". In addition to the heartbeat sensor, an acceleration sensor may be mounted on the sensor terminal 10. As an example, a triaxial acceleration sensor may be adopted as the acceleration sensor. The time-series data of the triaxial acceleration sensed using the acceleration sensor as described above, that is, change in acceleration in the vertical, lateral, and front-and-back directions of the sensor terminal 10 is used for detecting the exercise state of the user using the sensor terminal 10, such as walking, ascent and descent, and running. In this manner, the time-series data of acceleration contributes to removal of the exercise period in which the exercise state continues from the time-series data of the heart rate. In the following explanation, the time-series data of acceleration sensed using the acceleration sensor may also be referred to as "acceleration data". The heartbeat sensor and the acceleration sensor are illustrated as examples; however, the examples do not prevent mounting other sensors such as a gyro sensor and a global positioning system (GPS) receiver.

When a heartbeat sensor is mounted as described above, an attachment-type heartbeat sensor attached to the living body region of the user, such as the chest, the arm, and the wrist, may be adopted. For example, the pulse with a photoelectric pulse wave sensor may be adopted. In such a case, a heartbeat sensor dedicated for health care may be mounted, or a heartbeat sensor mounted on the wearable gadget may be applied. In addition, a heartbeat sensor detecting the heart rate is not necessarily mounted on the sensor terminal 10; however, an electrocardiograph sensor detecting an electrocardiographic signal may be mounted on the sensor terminal 10. The heartbeat sensor does not have to be an attachment type. For example, the heart rate may be detected from time-series change in brightness relating to an image obtained by imaging part of the living body of the user at predetermined sampling cycles, or a radio frequency (RF) motion sensor may be used to detect the Doppler frequency accompanying the pulsation, to achieve detection of the heart rate in a state of not contacting the living body of the user.

The feature quantities determined from the heart rate data described above and the acceleration data described above are transmitted from the sensor terminal 10 to the server apparatus 100, in a state of being associated with the user's identification information, such as the machine name or the serial number of the sensor terminal 10. The feature quantities are calculated with the sensor terminal 10 as described above, to reduce the data quantity transmitted to the network and prevent a situation in which the heart rate data being personal information is disclosed to the third party during transmission. This example illustrates the case where the feature quantities are transmitted from the sensor terminal 10 to the server apparatus 100; however, the sensor terminal 10 may transmit the heart rate data to cause the server apparatus 100 to calculate the feature quantities.

The server apparatus 100 is a computer providing the health care support services described above.

As an embodiment, the server apparatus 100 may be implemented by installing a meal time estimation program achieving the health care support services described above as package software or online software in a desired computer. For example, the server apparatus 100 may be implemented as a web server providing the health care support services described above, or implemented as a cloud providing the health care support services described above by outsourcing.

For example, the server apparatus 100 estimates the meal time of the user of the sensor terminal 10, using the feature quantities received from the sensor terminal 10. Thereafter, the server apparatus 100 records the meal time, generates and outputs a list of the meal time periods for a predetermined period, such as one week, based on the meal time recorded in the past, and performs analysis relating to the dietary habits or diet based on the meal time recorded in the past, to output various advices. For example, when the sensor terminal 10 includes an output device such as a display device, a sound output device, and a printing device, various pieces of information described above can be output through the output device of the sensor terminal 10. The output destination of the information is not necessarily the sensor terminal 10, but may be another terminal device used by the user, or a terminal device used by the persons concerned, such as the relatives of the user and the person in charge of medical or nursing care. In this manner, the health care support services described above are achieved.

The following explanation illustrates the feature quantities used for estimating the meal time in the health care support system 1 according to the present embodiment, and thereafter illustrates the functional structures of the devices included in the health care support system 1 according to the present embodiment.

Feature Quantities

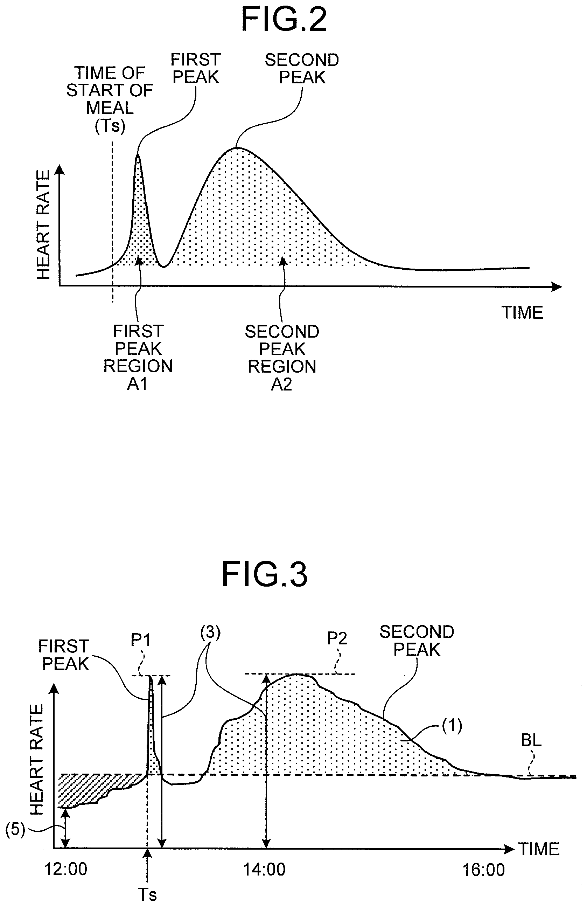

FIG. 2 is a diagram illustrating an example of heart rate data. FIG. 2 graphs change in heart rate before and after the start of the meal, as an example. The vertical axis illustrated in FIG. 2 indicates the heart rate per unit time, and the horizontal axis indicates the elapsed time (time) from the time direct before the start of the meal.

As illustrated in FIG. 2, the change in heart rate occurring with the meal after the start of the meal includes two peaks, in each of which the heart rate rises (increases) and lowers (decreases) with a lapse of time. Specifically, with a lapse of time from the meal start time Ts, a first peak and a second peak appear. The first peak is a peak of the heart rate change appearing first after the start of the meal. The second peak is a peak of the heart rate change appearing following the first peak. In the following explanation, a predetermined region including the waveform of the part of the first peak may also be referred to as "first peak region A1", and a predetermined region including the waveform of the part of the second peak may also be referred to as "second peak region A2".

Among the peaks, the "first peak" is increase in heartbeat accompanying the dietary action, and estimated to be, for example, increase in heart rate caused by vermicular movement of the esophagus. The "second peak" is estimated to be, for example, increase in heart rate caused by digestive action in digestive organs (such as the stomach and intestines) for the ingesta ingested by dietary action, that is, food.

As described above, after the meal, the second peak appears after the first peak appears, and the second peak tends to extend over a longer period of time than the first peak. To distinguish increase in heartbeat caused by meal with such tendency from increase in heartbeat caused by other causes, the feature quantities relating to the first peak and the second peak are defined as illustrated in the following items (1) to (5).

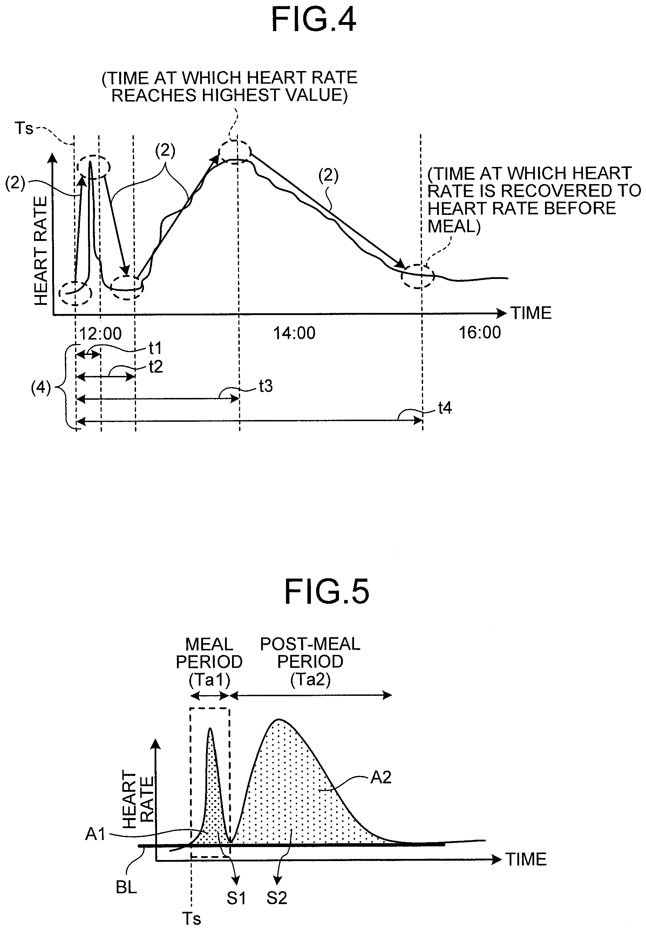

FIG. 3 and FIG. 4 are diagrams illustrating examples of heart rate data. Each of FIG. 3 and FIG. 4 graphs change in heart rate before and after start of the meal. The vertical axis illustrated in FIG. 3 and FIG. 4 indicates the heart rate per unit time, and the horizontal axis indicates the elapsed time (time) from the time directly before the start of the meal. Among them, FIG. 3 illustrates feature quantities (1), (3), and (5), and FIG. 4 illustrates feature quantities (2) and (4). The symbol "BL" illustrated in FIG. 3 indicates the baseline of the heart rate. The baseline BL is a reference value to determine the rise in the heart rate caused by meal. This is used for suppressing deterioration in the estimation accuracy of the meal time due to variations in heart rate among eating scenes, by determining the feature quantities using the rise in heart rate instead of the absolute value of the heart rate, because the heart rate does not always have the same value in each of the scenes in which the user have a meal. For example, the heart rate at the meal start time Ts can be used as the value of the heart rate of the baseline. Other examples of the value of the heart rate of the baseline include the average value of the user's heart rate for a predetermined period, such as 30 minutes and one hour before start of the meal, the heart rate at the meal start time Ts, and the minimum heart rate between the first peak and the second peak.

For example, the feature quantities indicating the likelihood that change in heart rate are caused by a meal can be defined based on five viewpoints of (1) area, (2) velocity, (3) amplitude, (4) time, and (5) pre-meal state. The five feature quantities are illustrated herein; however, not all the feature quantities are necessarily used for estimation of the meal time. The meal time can be estimated by using at least one of the five feature quantities.

Specifically, as illustrated in FIG. 3, the area of the first peak region A1 and the area of the second peak region A2 are defined as the feature quantities (1). In addition, as illustrated in FIG. 4, the increase velocity of the heart rate until the heart rate reaches the first peak, the increase velocity of the heart rate until the heart rate reaches the second peak, the recovery velocity of the heart rate from the first peak, and the recovery rate of the heart rate from the second peak are defined as the feature quantities (2). As illustrated in FIG. 3, the maximum heart rate P1 having the heart rate with the maximum value in the waveform forming the first peak, and the maximum heart rate P2 having the heart rate with the maximum value in the waveform forming the second peak are defined as the feature quantities (3). In addition, as illustrated in FIG. 4, when the meal start time Ts is the start point, the elapsed time t1 with an end point being the time at which the maximum heart rate P1 is measured, the elapsed time t2 with an end point being the time at which the heart rate through the maximum heart rate P1 is recovered to the predetermined value, the elapsed time t3 with an end point being the time at which the maximum heart rate P2 is measured, and the elapsed time t4 with an end point being the time at which the heart rate through the maximum heart rate P2 is recovered to the predetermined value are defined as the feature quantities (4). As illustrated in FIG. 3, the pre-meal heart rate having the lowest value among the heart rate measured before the meal start time Ts, and the area of the region formed of the rise of the heart rate from the pre-meal heart rate to the baseline BL in a section before the meal start time Ts are defined as the feature quantities (5). In the following explanation, the increase velocity described above and the recovery velocity described above may also be collectively referred to as "response velocity".

The following is explanation of methods for calculating the five feature quantities, that is, (1) area, (2) velocity, (3) amplitude, (4) time, and (5) pre-meal state.

(1) Area

FIG. 5 is a diagram illustrating an example of a method for calculating the feature quantities (1). FIG. 5 graphs change in heart rate before and after start of the meal. The vertical axis in FIG. 5 indicates the heart rate per unit time, and the horizontal axis indicates the elapsed time (time) from the time directly before the start of the meal.

In the feature quantities (1) described above, the area S1 of the first peak region A1 can be determined by summing the rises of the heart rate from the baseline BL in the meal period Ta1, as illustrated in FIG. 5. This example illustrates the case of calculating the area by summing the rises of the heart rate; however, the area may be determined by calculating the average value of the rises of the heart rate.

For example, as the meal period Ta1, a section with a start point being the meal start time Ts and an end point being the time at which the heart rate is recovered to the baseline BL through the first peak is set, as illustrated with a broken line in FIG. 5. Fluctuations in heart rate around the first peak are influenced by the user's vermicular movement. For example, when the vermicular movement is prolonged, there may be the case where the heart rate is not recovered to the baseline BL after going through the first peak. For this reason, the end point of the meal period Ta1 may be set to the time with the minimum heart rate in the section between the first peak and the second peak. The end point of the meal period Ta1 may be the time at which a certain period, such as 10 minutes and 20 minutes, has elapsed from the meal start time Ts. As an example, the certain period can be determined based on the statistical value of the time taken for vermicular movement.

By contrast, the area S2 of the second peak region A2 can be determined by, for example, summing the rises of the heart rate from the baseline BL in the post meal period Ta2.

For example, as the post-meal period Ta2, a section with a start point being the end point of the meal period Ta1 of the first peak region A1 or the time after the end point, and an end point being the time at which the heart rate is recovered to the baseline BL after going through the second peak is set. Fluctuations in heart rate in the second peak are influenced by the digestive action in the digestive organs. For example, when the digestive action is prolonged, there may be the case where the heart rate is not recovered to the baseline BL after going through the second peak. For this reason, the end point of the post-meal period Ta2 may be the time at which a certain period, such as 240 minutes, has elapsed from the start point of the post-meal period Ta2. As an example, the certain period can be determined based on the statistical value of the time taken for digestive action.

This example illustrates the case where the area S1 of the first peak region A1 and the area S2 of the second peak region A2 are calculated from the rises of the heart rate from the baseline BL; however, the structure is not limited thereto. The areas may be calculated from the rises from the pre-meal heart rate.

(2) Velocity

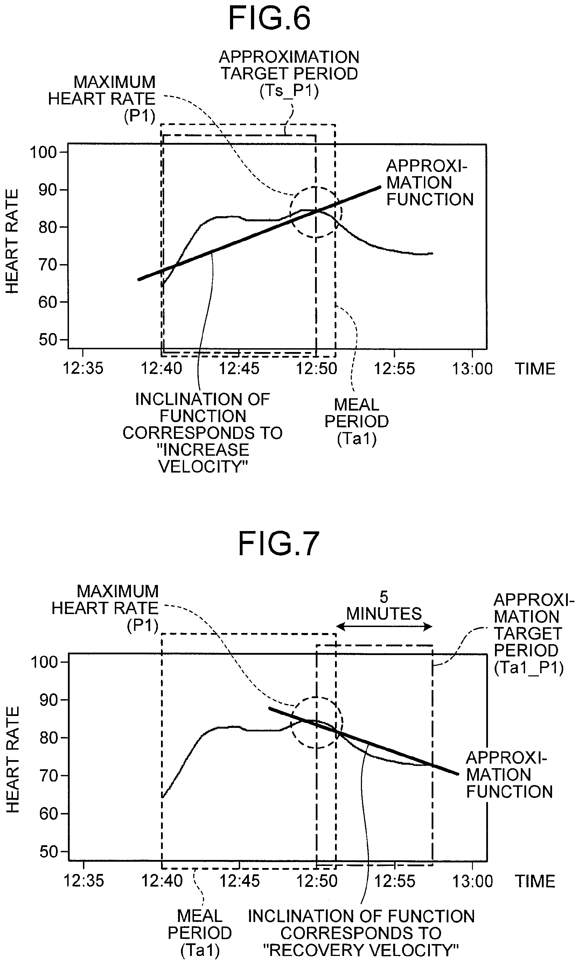

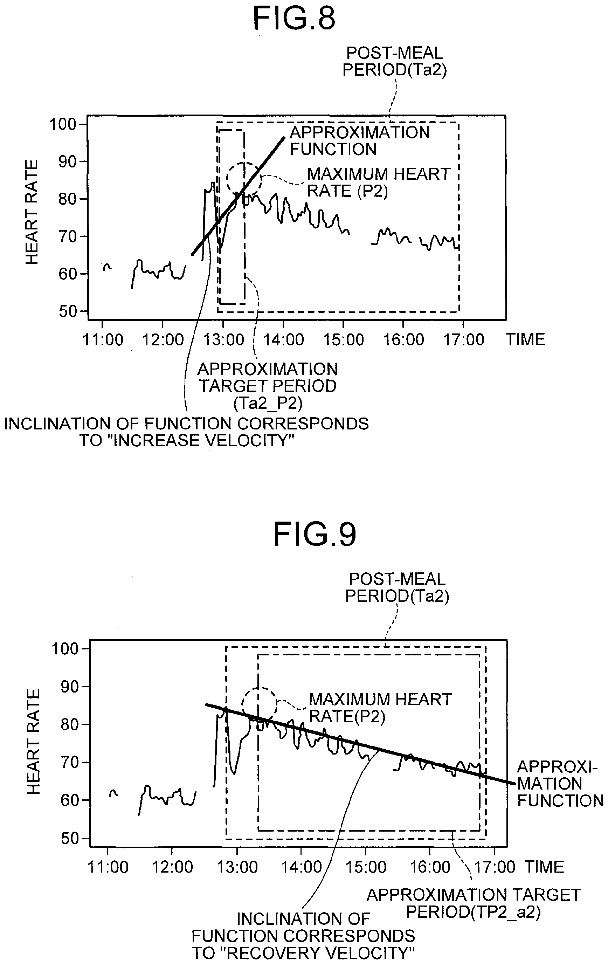

FIG. 6 to FIG. 9 are diagrams illustrating an example of a method for calculating the feature quantities (2). The vertical axis of each of FIG. 6 to FIG. 9 indicates the heart rate per unit time, and the horizontal axis indicates the elapsed time (time) from the time directly before the start of the meal. Among them, FIG. 6 and FIG. 7 illustrate a closed-up waveform around the first peak among the waveform of the heart rate measured after start of the meal, while FIG. 8 and FIG. 9 illustrate the closed-up waveform around the second peak. In FIG. 6 and FIG. 7, a section corresponding to the meal period Ta1 in the waveform of the heart rate is enclosed with a broken line, while a section corresponding to the post-meal period Ta2 in the waveform of the heart rate is enclosed with a broken line in FIG. 8 and FIG. 9.

Among the feature quantities (2) described above, the increase velocity of the heart rate until the heart rate reaches the first peak can be calculated from increase change in heart rate observed in a period with, for example, a start point being the meal start time Ts and an end point being the time at which the maximum heart rate P1 is measured in the meal period Ta1. Specifically, as illustrated in FIG. 6, the period from the meal start time Ts to the time at which the maximum heart rate P1 is measured is set as "approximation target period Ts_P1", and function approximation by linear function or the like is performed on a data string of the heart rate included in the approximation target period Ts_P1 indicated with a dashed line in FIG. 6. In this manner, the inclination of the approximation expression approximated to transition of the heart rate of the approximation target period Ts_P1 is determined. Because such inclination is equivalent to the increase velocity of the heart rate in the approximation target period Ts_P1, the inclination can be used as the increase velocity of the first peak.

In the same manner as above, the recovery velocity of the heart rate from the first peak can also be calculated. For example, the recovery change of the heart rate from the time at which the maximum heart rate P1 is measured in the meal period Ta1 to the time until a certain period is elapsed after end of the meal can be calculated as the velocity. A period with the unit of minutes such as five minutes can be adopted as an example of the certain period after end of the meal. Specifically, as illustrated in FIG. 7, the period from the time at which the maximum heart rate P1 is measured to the time until the certain period is elapsed after end of the meal is set as "approximation target period Ta1_P1". Under such setting, function approximation by linear function or the like is performed on a data string of the heart rate included in the approximation target period Ta1_P1 indicated with a dashed line in FIG. 7. In this manner, the inclination of the approximation expression approximated to transition of the heart rate of the approximation target period Ts_P1 is determined. Because such inclination is equivalent to the recovery velocity of the heart rate in the approximation target period Ts_P1, the inclination can be used as the recovery velocity of the first peak.

In addition, the increase velocity of the heart rate until the heart rate reaches the second peak can be calculated from increase change of the heart rate observed in a period with a start point being the start time of the post-meal period Ta2, that is, the end time of the meal period Ta1, and an end point being the time at which the maximum heart rate P2 is measured in the post-meal period Ta2, for example. Specifically, as illustrated in FIG. 8, the period from the start time of the post-meal period Ta2 to the time at which the maximum heart rate P2 is measured is set as the "approximation target period Ta2_P2", and function approximation by linear function or the like is performed on a data string of the heart rate included in the approximation target period Ta2_P2 indicated with a dashed line in FIG. 8. In this manner, the inclination of the approximation expression approximated to transition of the heart rate of the approximation target period Ta2_P2 is determined. Because such inclination is equivalent to the increase velocity of the heart rate in the approximation target period Ta2_P2, the inclination can be used as the increase velocity of the second peak.

In addition, the recovery velocity of the heart rate from the second peak can be calculated as velocity being recovery change of the heart rate from the time at which the maximum heart rate P2 is measured in the post-meal period Ta2 to the time at which digestive action with the digestive organ is ended, for example. For example, the end time of the post-meal period Ta2 can be adopted as an example of the time at which the digestive action is ended. Specifically, as illustrated in FIG. 9, the period from the time at which the maximum heart rate P2 is measured in the post-meal period Ta2 to the end time of the post-meal period Ta2 is set as the "approximation target period TP2_a2". Under such setting, function approximation by linear function or the like is performed on a data string of the heart rate included in the approximation target period TP2_a2 indicated with a dashed line in FIG. 9. In this manner, the inclination of the approximation expression approximated to transition of the heart rate of the approximation target period TP2_a2 is determined. Because such inclination is equivalent to the recovery velocity of the heart rate in the approximation target period TP2_a2, the inclination can be used as the recovery velocity of the second peak.

This explanation illustrates four response velocities including two increase velocities and two recovery velocities; however, not all the response velocities are necessarily used for estimation of the meal time. For example, either of the two increase velocities and the two recovery velocities among the four response velocities may be used for estimation of the meal time.

(3) Amplitude

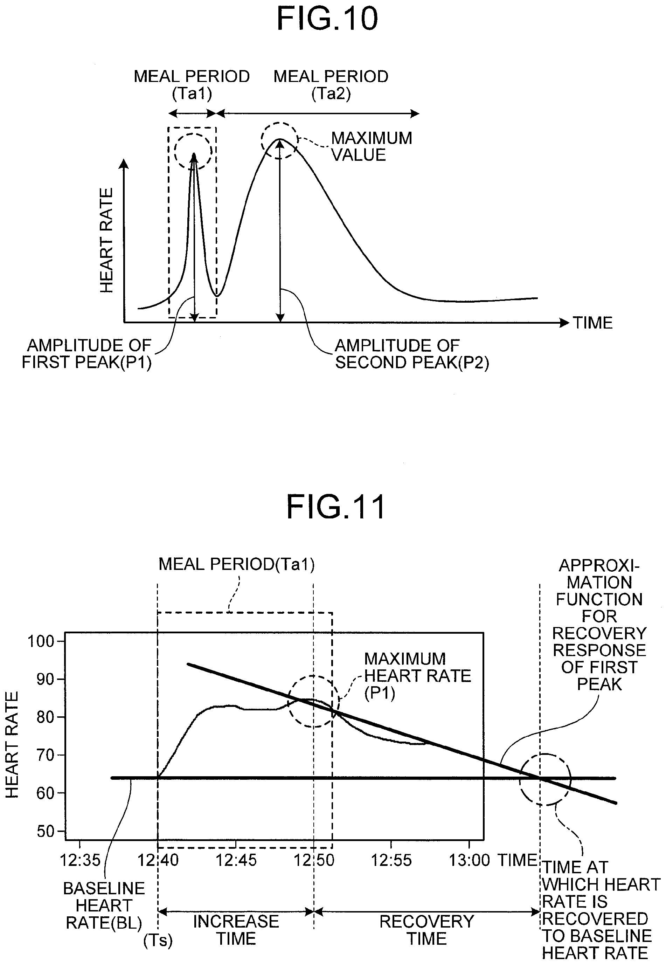

FIG. 10 is a diagram illustrating an example of a method for calculating the feature quantities (3). FIG. 10 graphs change in heart rate before and after start of the meal. The vertical axis illustrated in FIG. 10 indicates the heart rate per unit time, and the horizontal axis indicates the elapsed time (time) from the time directly before the start of the meal.

Among the feature quantities (3) described above, the amplitude of the first peak can be derived by extracting the maximum heart rate P1 being the maximum heart rate in the heart rates measured in the meal period Ta1 from the heart rate data, as illustrated in FIG. 10. In the same manner, the amplitude of the second peak can be derived by extracting the maximum heart rate P2 being the maximum heart rate in the heart rates measured in the post-meal period Ta2 and the heart rate data.

The amplitude of the first peak and the amplitude of the second peak are not necessarily the absolute values from zero; however, the maximum value of the rise of the heart rate may be calculated in each of the first peak and the second peak by subtracting the heart rate of the baseline BL from the maximum heart rate P1 or the maximum heart rate P2. Because the second peak is influenced by digestive action, for example, the time period in which the maximum heart rate P2 is detected in the post-meal period Ta2 may be limited to the time period in which the digestive action is supposed to become most active. For example, an example of the time period in which the digestive action is supposed to become most active is a period of 30 minutes to 80 minutes after the meal start time Ts.

(4) Time

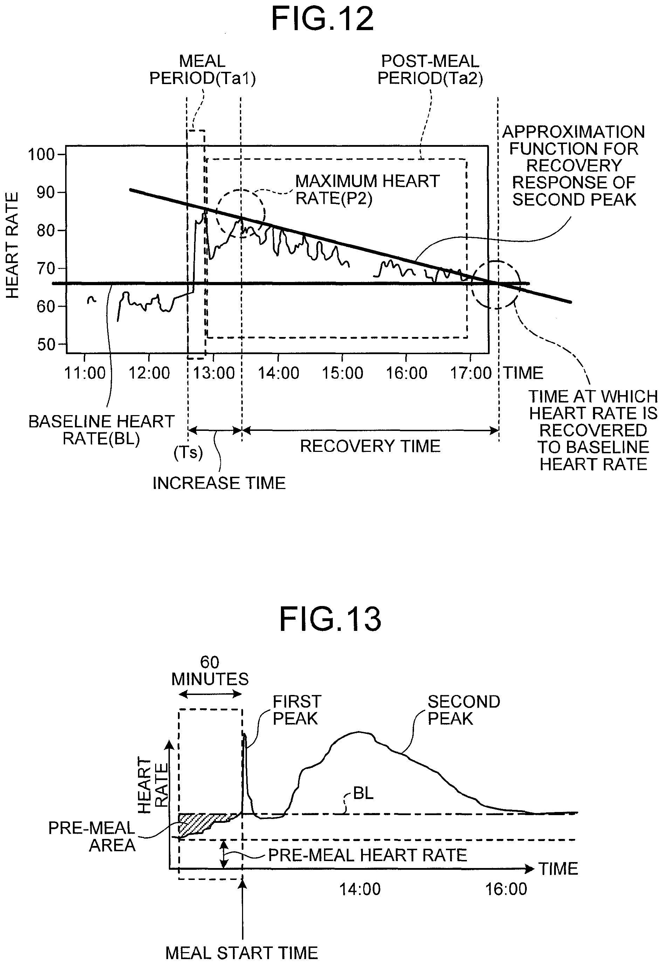

FIG. 11 and FIG. 12 are diagrams illustrating an example of a method for calculating the feature quantities (4). FIG. 11 and FIG. 12 graph change in heart rate before and after start of the meal. The vertical axis in FIG. 11 and FIG. 12 indicates the heart rate per unit time, and the horizontal axis indicates the elapsed time (time) from the time directly before the start of the meal.

Among them, FIG. 11 illustrates the closed-up waveform around the first peak among the waveform of the heart rate measured after the start of the meal. In addition, in FIG. 11, a section corresponding to the meal period Ta1 in the waveform of the heart rate is enclosed with a rectangular broken line, and the part of the graph having the value of the maximum heart rate P1 serving as the amplitude of the first peak in the waveform of the heart rate is enclosed with a circular broken line.

Among the response time defined as the feature quantities (4) described above, the increase time of the heart rate to the first peak can be calculated as the time from the start time of the meal period Ta1, that is, the meal start time Ts, to the time at which the maximum heart rate P1 is measured in the meal period Ta1, as illustrated in FIG. 11. In addition, the recovery time of the heart rate from the first peak can be calculated as, for example, the time from the time at which the maximum heart rate P1 is measured in the meal period Ta1 to the time at which the heart rate is recovered to the heart rate of the baseline BL.

Because the heart rate measured around the first peak is influenced by the vermicular movement of the esophagus as described above, there may be the case where the heart rate is not recovered to the baseline BL until the heart rate reaches the second peak.

For this reason, the recovery time of the heart rate from the first peak can also be determined by using the approximation expression explained and illustrated in FIG. 7 with respect to the feature quantities (2) described above. The recovery velocity of the first peak illustrated in FIG. 7 is calculated as the inclination of the approximation function approximated from the recovery change of the heart rate occurring after the heart rate reaches the first peak. For this reason, as illustrated in FIG. 11, it can be calculated by determining the elapsed time until the heart rate transitioning with the inclination of the approximation function reaches the heart rate of the baseline BL. For example, the time of the intersection point between the approximation function indicating the recovery velocity after measurement of the maximum heart rate P1 and the baseline BL is determined, that is, the time of the point enclosed with a circular dashed line in FIG. 11 is determined. Specifically, the time of the intersection point is determined by substituting the heart rate of the baseline BL for the variable of the heart rate among the two variables of the time and the heart rate included in the approximation function, and the recovery time of the heart rate from the first peak can be determined by subtracting the time at which the maximum heart rate P1 is measured from the time of the intersection point. The recovery time of the heart rate from the first peak can also be calculated by determining a difference between the maximum heart rate P1 and the heart rate of the baseline BL, and dividing the difference between them by the inclination of the approximation function.

By contrast, in FIG. 12, a section corresponding to the meal period Ta1 and the post-meal period Ta2 in the waveform of the heart rate is enclosed with a rectangular broken line, and the part of the graph having the value of the maximum heart rate P2 serving as the amplitude of the second peak in the waveform of the heart rate is enclosed with a circular broken line.

Among the response times defined as the feature quantities (4) described above, the increase time of the heart rate until the second peak can be calculated as the time from the start time of the meal period Ta1, that is, from the meal start time Ts, to the time at which the maximum heart rate P2 is measured in the post-meal period Ta2, as illustrated in FIG. 12. In addition, the recovery time of the heart rate from the second peak can be calculated as the time from, for example, the time at which the maximum heart rate P2 is measured in the post-meal period Ta2 to the time at which the heart rate is recovered to the heart rate of the baseline BL.

Because the heart rate measured around the second peak is influenced by the digestive action as described above, there may be the case where the heart rate is not recovered to the baseline BL after the heart rate reaches the second peak.

For this reason, the recovery time of the heart rate from the second peak can also be determined by using the approximation expression explained and illustrated in FIG. 9 with respect to the feature quantities (2) described above. The recovery velocity of the second peak illustrated in FIG. 9 is calculated as the inclination of the approximation function approximated from the recovery change of the heart rate occurring after the heart rate reaches the second peak. For this reason, as illustrated in FIG. 12, it can be calculated by determining the elapsed time until the heart rate transitioning with the inclination of the approximation function reaches the heart rate of the baseline BL. For example, the time of the intersection point between the approximation function indicating the recovery velocity after measurement of the maximum heart rate P2 and the baseline BL is determined, that is, the time of the point enclosed with a circular dashed line in FIG. 12 is determined. Specifically, the time of the intersection point is determined by substituting the heart rate of the baseline BL for the variable of the heart rate among the two variables of the time and the heart rate included in the approximation function, and the recovery time of the heart rate from the second peak can be determined by subtracting the time at which the maximum heart rate P2 is measured from the time of the intersection point. The recovery time of the heart rate from the second peak can also be calculated by determining a difference between the maximum heart rate P2 and the heart rate of the baseline BL, and dividing the difference between them by the inclination of the approximation function.

(5) Pre-meal state

FIG. 13 is a diagram illustrating an example of a method for calculating the feature quantities (5). FIG. 13 graphs change in heart rate before and after start of the meal. The vertical axis illustrated in FIG. 13 indicates the heart rate per unit time, and the horizontal axis indicates the elapsed time (time) from the time directly before the start of the meal.

As illustrated in FIG. 13, among the feature quantities (5) as described above, the pre-meal heart rate can be derived by extracting the heart rate having the lowest value among the heart rates measured before the meal start time Ts from the heart rate data. At the time, a period such as 60 minutes can be adopted as an example of a section retroactive to the time before the meal start time Ts. In addition, the pre-meal area can be determined by summing the rises of the heart rate from the pre-meal heart rate in the section before the meal start time Ts. This explanation illustrates the case of calculating the area by summing the rises of the heart rate; however, the area may be determined by calculating the average value of the rises of the heart rate.

By using the fourteen feature quantities of five types for estimation of the meal time, increase in heart rate caused by meal and increase in heart rate caused by the other causes can be distinguished. Accordingly, this structure suppresses decrease in estimation accuracy of the meal time. In addition, because information other than the heart rate data is not necessarily used for calculation of the feature quantities described above, the feature quantities can be calculated without using sensors other than the heartbeat sensor. Specifically, on the premise that the heartbeat sensor is not always used alone, the estimation accuracy of the meal time can be maintained with the heartbeat sensor alone.

In addition, at least the following two reasons can be mentioned as factors enabling suppressing of decrease in estimation accuracy of the meal time with the heartbeat sensor alone. The first reason is using the feature quantities enabling estimation as to whether change in heart rate is caused by meal or other causes, including change in heart rate caused by digestive action, that is, the second peak region A2, for estimation of the meal time. The second reason is using the feature quantities indicating recovery change of the heart rate, that is, the recovery velocity and the recovery time for estimation of the meal time. In the conventional meal determination described above, it is not supposed to estimate the meal time using these two points, or using the other feature quantities. Accordingly, the method for estimating the meal time according to the present embodiment enables exhibition of an advantageous effect that is not able to be accomplished with the conventional meal determination described above, that is, suppression of decrease in estimation accuracy of the meal time with the heartbeat sensor alone.

Configuration of the sensor terminal 10

The following is explanation of a functional structure of the sensor terminal 10 according to the present embodiment. As illustrated in FIG. 1, the sensor terminal 10 includes a heart rate data acquisition unit 11, an acceleration data acquisition unit 12, an exercise period determination unit 13, a noise heart rate removal unit 14, a window data preparation unit 15, a feature quantity calculator 16, an eating action determination unit 17, and a communication I/F (interface) unit 18. The sensor terminal 10 may include functional units included in a known computer other than the functional units illustrated in FIG. 1. For example, when a terminal device dedicated for health care, a wearable gadget or a mobile terminal device is executed as the sensor terminal 10, it is needless to say that each of the devices can be equipped with hardware and software usually mounted thereon.

The heart rate data acquisition unit 11 is a processor acquiring the heart rate data described above.

As an embodiment, the heart rate data acquisition unit 11 controls the heartbeat sensor to cause the heartbeat sensor to sense the heart rate at predetermined sampling cycles. In this manner, the heart rate data acquisition unit 11 acquires time-series data of the heart rate sensed by the heartbeat sensor for each sampling point as heart rate data. As an example, data in which items such as the time and the heart rate are associated can be adopted as the heart rate data. The term "time" herein may be system time locally managed on the sensor terminal 10, such as the elapsed time from a desired start point, or the time expressed on the calendar such as year, month, day, time, minute, and second. The term "heart rate" is expressed as the heart rate per unit time. For example, when the unit time is one minute, the heart rate is expressed with beats per minute (bpm) or the like. When the unit time is one second, the heart rate is expressed with Hz.

The heart rate data acquired as described above may be output to the functional unit of the following stage each time the heart rate is sensed, or output to the functional unit of the following stage after the heart rates are accumulated for a predetermined period, such as twelve hours and one day.

The acceleration data acquisition unit 12 is a processor acquiring the acceleration data described above.

As an embodiment, the acceleration data acquisition unit 12 controls the acceleration sensor to cause the acceleration sensor to sense accelerations of the three axes, that is, the vertical, the lateral, and the front-and-back accelerations at predetermined sampling cycles. In this manner, the acceleration data acquisition unit 12 acquires time-series data of the vertical, the lateral, and the front-and-back accelerations sensed by the acceleration sensor for each sampling point, as acceleration data. As an example, data in which items such as the time and the acceleration are associated can be adopted as the acceleration data. The term "time" herein may be system time locally managed on the sensor terminal 10, such as the elapsed time from a desired start point, or the time expressed on the calendar such as year, month, day, time, minute, and second, in the same manner as the heart rate data described above. In addition, the "acceleration" may include triaxial accelerations, that is, the vertical, the lateral, and the front-and-back accelerations. For example, when acceleration of part of directions among the triaxial accelerations is used by the functional unit of the following stage, the acceleration of the direction that is not used in the functional unit of the following stage may be removed from the acceleration data. The acceleration sensor may be caused to adopt the same sampling cycles as those of the heartbeat sensor, or adopt different sampling cycles.

The acceleration data acquired as described above may be output to the functional unit of the following stage each time the acceleration is sensed, or output to the functional unit of the following stage after the heart accelerations are accumulated for a predetermined period, such as twelve hours and one day.

The exercise period determination unit 13 is a processor determining the exercise period. The "exercise period" herein indicates a period in which exercise such as walking, running, and ascent and descent of stairs is supposed to be performed. For example, the acceleration data acquired with the acceleration data acquisition unit 12 is used for determination of the exercise period.

As an embodiment, the exercise period determination unit 13 uses at least the acceleration of the vertical direction, that is, the gravity direction, among the accelerations included in the acceleration data acquired with the acceleration data acquisition unit 12, for determination of the exercise period described above. The acceleration in the gravity direction is used as described above, because the acceleration changes with a specific pattern and the pattern periodically appears, when exercise such as walking, running, and ascent and descent of stairs is performed.

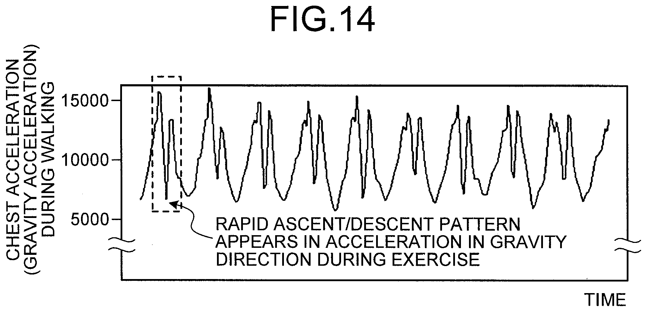

FIG. 14 is a diagram illustrating an example of the acceleration data. FIG. 14 illustrates an extract of change of the acceleration in the vertical direction among the accelerations sensed by the acceleration sensor in exercise. The vertical axis illustrated in FIG. 14 indicates the acceleration in the gravity direction, and the horizontal axis indicates the time. FIG. 14 also illustrates an example of a pattern of change in acceleration measured in exercise, enclosed with a broken line in a rectangular shape. Suppose that the acceleration in the gravity direction illustrated in FIG. 14 is measured in a state where the sensor terminal 10 equipped with the acceleration sensor is attached to the chest of the user.

As illustrated in a state being enclosed with a broken line in FIG. 14, in exercise such as walking, running, and ascent and descent of stairs, because reaction force is received from the ground or the like when the user kicks the ground and lands with the foot, change of increase and decrease appears in the acceleration in the gravity direction in a short period. In addition, as illustrated in FIG. 14, change of increase and decrease relating to the acceleration in the gravity direction periodically appears each time the load is removed from the ground and the user's foot lands the ground.

For this reason, as an example, the exercise period determination unit 13 detects the increase and decrease pattern illustrated in FIG. 14, from the acceleration in the gravity direction acquired with the acceleration data acquisition unit 12. Thereafter, the exercise period determination unit 13 determines whether the intervals at which the pattern appears fall within a predetermined period. When the intervals at which the pattern appears fall within a predetermined period, a section in which the pattern repeatedly appears in the predetermined period is determined to be the exercise period. To determine the exercise period described above, as an example, the user performs exercise action in a state where the sensor terminal 10 is attached to the user, to experimentally collect change in acceleration accompanying the exercise action. This operation enables setting a threshold to distinguish increase and decrease based on the degree of increase and decrease forming the pattern described above. In addition, the predetermined period can be determined based on experimentally appearing values also with respect to the intervals at which the pattern appears. The exercise period described above can be expressed with the start time and the duration time of the exercise period, or expressed as the exercise start time and the exercise end time.

The noise heart rate removal unit 14 is a processor removing a section estimated to be a section in which the heart rate changes due to noise other than meal, from the heart rate data described above.

As an embodiment, the noise heart rate removal unit 14 specifies increase change in heart rate caused by exercise action, and noise change in heart rate irregularly occurring in the meal period and the post-meal period, which influence time-series change in the heart rate acquired with the heart rate data acquisition unit 11. The noise heart rate removal unit 14 removes heart rate data detected with increase change in heart rate caused by specified exercise action, and noise change in heart rate irregularly occurring in the meal period and the post-meal period, from the time series of the heart rate acquired with the heart rate data acquisition unit 11.

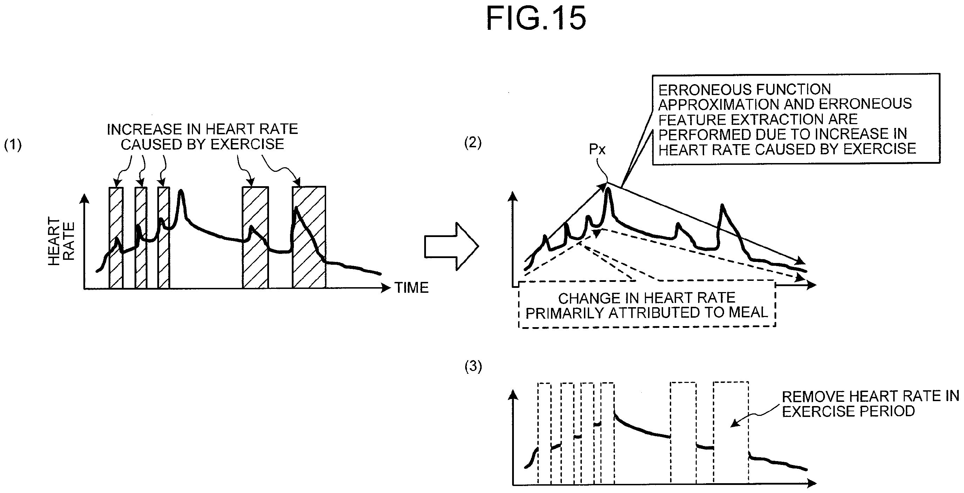

FIG. 15 is a diagram illustrating an example of a method for removing the noise heart rate. FIG. 15 graphs heart rate data including change in heart rate accompanying meal and change in heart rate accompanying exercise. The vertical axis indicates the heart rate per unit time, and the horizontal axis indicates the elapsed time (time) from the time directly before the start of the meal.

(1) of FIG. 15 illustrates sections corresponding to exercise periods in the heart rate data with portions filled with lines going down toward the right. As described above, when the heart rate data includes change in heart rate accompanying exercise, increase change in heart rate, and recovery change of the increase heart rate with a lapse of time appear in the exercise period. By contrast, because the digestive action in the post-meal period and the like has an influence on the heart rate over a longer period than that of eating and vermicular movement of the esophagus, when exercise is performed after the meal, change in heart rate accompanying exercise may overlap change in heart rate accompanying the digestive action. In such a case, change in heart rate accompanying exercise may have an adverse influence on estimation of the meal time.

(2) of FIG. 15 illustrates change in heart rate without increase change and recovery change in heart rate caused by exercise, that is, change in heart rate caused by the meal alone, with a broken line with an arrow. As illustrated in the drawing, when change in heart rate caused by exercise overlaps change in heart rate accompanying the meal, it may cause indexing of the feature quantities relating to the second peak, such as the amplitude, the increase velocity, and the recovery velocity, and the feature quantities such as the increase time and the recovery time, with larger values than those in the case without exercise. This may cause an adverse influence of change in heart rate accompanying exercise on estimation of the meal time.

For example, in the example illustrated in (2) of FIG. 15, the heart rate of Px is calculated as the amplitude of the second peak, that is, the maximum heart rate. In addition, as illustrated with a solid line with an arrow in FIG. 15, the feature quantities relating to the second peak, such as the increase velocity and the recovery velocity, and the increase time and the recovery time can be calculated from change in heart rate overlapping the increase change and the recovery change in heart rate caused by exercise.

As described above, to suppress an adverse influence of change in heart rate accompanying exercise on estimation of the meal time, the noise heart rate removal unit 14 removes the section corresponding to the exercise period determined with the exercise period determination unit 13, from the heart rate data acquired by the heart rate data acquisition unit 11.

In the operation, the noise heart rate removal unit 14 can remove, from the heart rate data, not only the section corresponding to the exercise period but also the section corresponding to the removal period by determining the removal period including recovery change of the heart rate after exercise by adding, to the exercise end time, a fixed period from the exercise end time until the heart rate increased by exercise is recovered, as an example. As an example, as the fixed period added to the exercise end time, a time period specific to the user may be set by performing experiments of measuring the recovery change after exercise, or a time period common to all the users may be set as the default value. When data of the section corresponding to the exercise period or the removal period is removed from the heart rate data, data of the part omitted by the removal may be interpolated, by executing linear interpolation, polynomial interpolation, spline interpolation, or the like.

(3) of FIG. 15 illustrates heart rate data obtained by removing the section corresponding to the removal period from the heart rate data illustrated in (1) of FIG. 15, and illustrates the removal periods enclosed with rectangular broken lines. As illustrated in (3) of FIG. 15, the sections corresponding to the removal periods are removed from the heart rate data, to enable calculation of the feature quantities of heart rate change accompanying the meal without influence of increase change and recovery change of the heart rate accompanying exercise.

As illustrated with the broken line with an arrow in (2) of FIG. 15, change in heart rate caused by the meal alone tends to transition along the relative lower limit of the heart rate. For this reason, for example, former and latter heart rates on the times series may be compared, to recognize change points at which a difference of the former and the latter heart rates is equal to or higher than a predetermined threshold, or equal to or lower than the predetermined threshold, and a section specified with the change points may be set as the removal period.

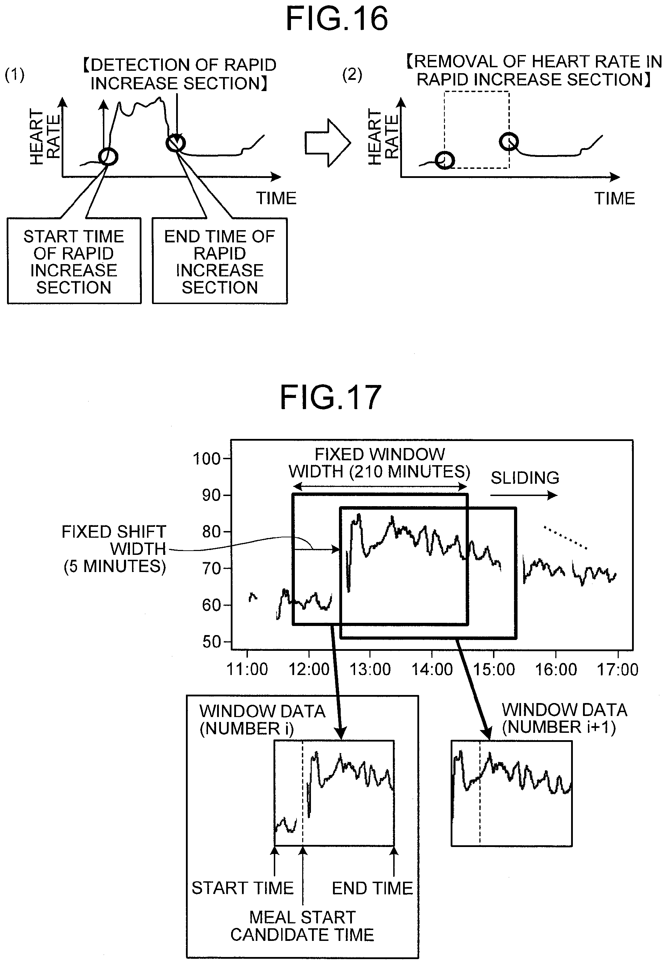

FIG. 16 is a diagram illustrating an example of a method for calculating the removal period based on change points. FIG. 16 illustrates a graph relating to change in heart rate. The vertical axis indicates the heart rate per unit time, and the horizontal axis indicates the elapsed time (time). (1) of FIG. 6 illustrates change points at which the heart rate rapidly increases and decreases. The change points are enclosed with circular solid lines.

For example, the noise heart rate removal unit 14 compares former and latter heart rates on the time series. In this manner, as illustrated in (1) of FIG. 16, the noise heart rate removal unit 14 specifies the change points at which a difference between the former and latter heart rates is equal to or higher than a predetermined threshold, or equal to or lower than the predetermined threshold. Thereafter, for example, the noise heart rate removal unit 14 provides identification numbers to identify the change points, to the specified change points. For example, when the former and latter heart rates on the time series have values equal to or higher than the predetermined value, the noise heart rate removal unit 14 stores the former heart rate on the time series and time information at which the heart rate is measured, in an internal memory (not illustrated), in association with the identification number provided to the change point. For example, also when the former and latter heart rates on the time series have values equal to or lower than the predetermined value, the noise heart rate removal unit 14 stores the latter heart rate on the time series and time information at which the heart rate is measured, in the internal memory, in association with the identification number provided to the change point.

Thereafter, the noise heart rate removal unit 14 specifies the removal period from the time information associated with the change point at which the difference value between the former and the latter heart rates is equal to or higher than the predetermined threshold, and the time information associated with the change point at which the difference value between the former and the latter heart rates is equal to or lower than the predetermined threshold. In the operation, there may be the case where change points at which the difference value between the heart rate values is equal to or higher than the predetermined threshold are successively detected on the time series. In the case where change points at which the difference value between the heart rate values is equal to or higher than the predetermined threshold successively appear on the time series, for example, the change point preceding on the time series, or the change point associated with a lower heart rate is set as the "start time" of the removal period. In the same manner, in the case where change points at which the difference value between the heart rate values is equal to or lower than the predetermined threshold successively appear on the time series, for example, the change point appearing later on the time series, or the change point associated with a lower heart rate is set as the "end time" of the removal period.

(2) of FIG. 16 illustrates a graph in which the heart rate corresponding to the removal period is removed from the heart rate data illustrated in (1) of FIG. 16. (2) of FIG. 16 illustrates the removal period in a state of being enclosed with a rectangular broken line. As illustrated in (2) of FIG. 16, removing the period corresponding to the removal period from the heart rate data enables calculation of the feature quantities of the heart rate change accompanying the meal, without influence of the increase change and the recovery change in heart rate of the removal period. For example, this structure enables removal of heart rate data serving as noise such as increase change and recovery change in heart rate occurring irregularly in the meal period and the post-meal period.

The window data preparation unit 15 is a processor preparing window data. The term "window data" herein indicates partial data obtained by extracting part of the heart rate data.

As an embodiment, the window data preparation unit 15 sets a window having a predetermined length of time, for example, 210 minutes, in the heart rate data after removal is performed with the noise heart rate removal unit 14. Thereafter, the window data preparation unit 15 extracts partial data corresponding to the section in which the window is set. Thereafter, the window data preparation unit 15 shifts the previously set window by a predetermined shift width, for example, five minutes. Thereafter, the window data preparation unit 15 extracts partial data corresponding to the shifted window. As described above, each time partial data is extracted with the window width, window data is prepared and output to the functional unit of the following stage.

FIG. 17 is a diagram illustrating an example of a method for preparing window data. FIG. 17 illustrates a graph relating to change in heart rate. The vertical axis indicates the heart rate per unit time, and the horizontal axis indicates the elapsed time (time). For example, when preparation of window data is started by matching the start time of the window with the start time of the heart rate data, sliding of the window is repeatedly performed until the end time of the window matches with the end time of the heart rate data. As an example, FIG. 17 illustrates window data i prepared from the ith set window, and window data i+1 prepared from the (i+1)th set window. For example, when the window data i+1 is prepared, the (i+1)th window is set by shifting the ith set window by a predetermined shift width, for example, five minutes. Thereafter, a section corresponding to the (i+1)th window in the heart rate data is extracted, to prepare the window data (i+1). Such sliding processing is repeatedly performed until the end time of the window matches with the end time of the heart rate data. This explanation illustrates the case of shifting the window in the advance direction of the time; however, it is needless to say that the window may be shifted in the past direction of the time.

The feature quantity calculator 16 is a processor calculating feature quantities of the heartbeat accompanying the meal.

As an embodiment, the feature quantity calculator 16 is capable of calculating the fourteen feature quantities of the five types described above, for each of pieces of window data prepared by the window data preparation unit 15. In the operation, as an example, the feature quantity calculator 16 calculates the feature quantities, with a pre-meal state being a predetermined period, such as a period for 60 minutes, from the start time of the window data illustrated in FIG. 17, and with "meal start time" set to a time after a predetermined period of time from the start time of the window data. For example, as the feature quantities (1) described above, the feature quantity calculator 16 is capable of calculating the area S1 of the first peak region A1, and the area S2 of the second peak region A2. As the feature quantities (2) described above, the feature quantity calculator 16 is capable of calculating the increase velocity of the heart rate until the heart rate reaches the first peak, recovery velocity of the heart rate from the first peak, the increase velocity of the heart rate until the heart rate reaches the second peak, and recovery velocity of the heart rate from the second peak. As the feature quantities (3) described above, the feature quantity calculator 16 is capable of calculating the amplitude of the first peak, and the amplitude of the second peak. As the feature quantities (4) described above, the feature quantity calculator 16 is capable of calculating the increase time of the heart rate until the first peak, recovery time of the heart rate from the first peak, the increase time of the heart rate until the second peak, and recovery time of the heart rate from the second peak. As the feature quantities (5) described above, the feature quantity calculator 16 is capable of calculating the pre-meal heart rate and the pre-meal area.

The eating action determination unit 17 is a processor determining eating action. The term "eating action" herein indicates action performed for the purpose of eating a meal, and includes, for example, action of carrying food to the mouth.

As an embodiment, the eating action determination unit 17 uses acceleration of at least one direction among the accelerations included in the acceleration data acquired with the acceleration data acquisition unit 12, for determination of the eating action. For example, when the sensor terminal 10 is attached to the chest, the acceleration in the front-and-back direction can be used. The acceleration in the front-and-back direction is used when the sensor terminal 10 is attached to the chest, because action of carrying food to the mouth appears as a reciprocal movement in the front-and-back direction.

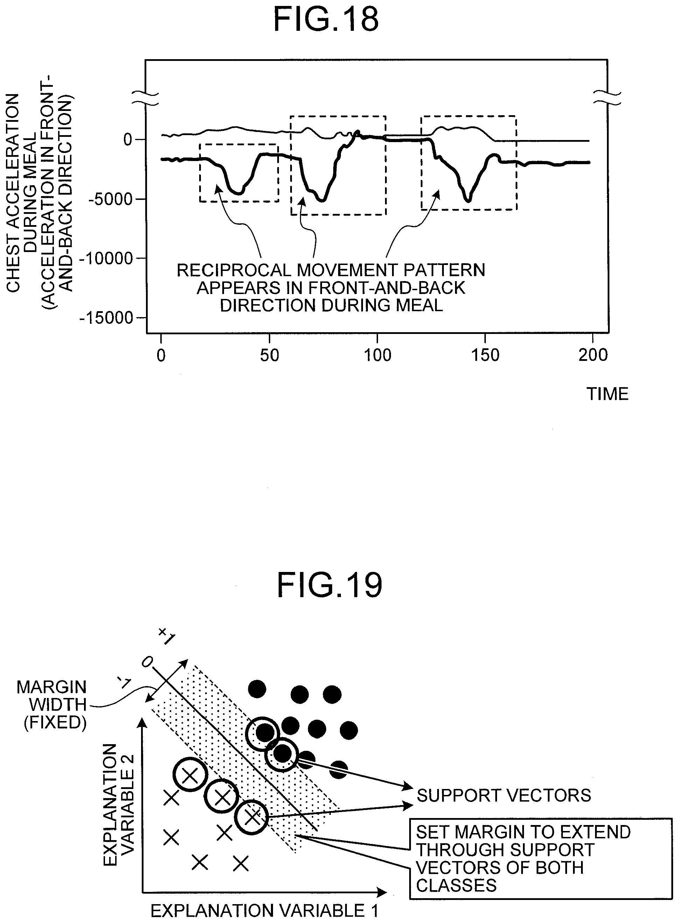

FIG. 18 is a diagram illustrating an example of acceleration data. FIG. 18 illustrates an extract of change of the acceleration in the front-and-back direction among the accelerations sensed by the acceleration sensor. The vertical axis illustrated in FIG. 18 indicates the acceleration in the front-and-back direction, and the horizontal axis indicates the time. FIG. 18 also illustrates an example of patterns of change in acceleration corresponding to the reciprocal movement in the front-and-back direction accompanying eating action, enclosed with broken lines in a rectangular shape. Suppose that the acceleration in the front-and-back direction illustrated in FIG. 18 is measured in a state where the sensor terminal 10 equipped with the acceleration sensor is attached to the chest of the user.

As illustrated in a state being enclosed with broken lines in FIG. 18, when the user takes a meal, because action to carry food to the lip portion is repeated, the following pattern appears in change in acceleration in the front-and-back direction. Specifically, a pattern in which the acceleration decreases and thereafter increases in linkage with a reciprocal movement accompanying the eating action repeatedly appears. Specifically, the acceleration changes with cycles of standstill, decrease, increase, standstill, decrease, increase, and standstill, for example.

For this reason, as an example, the eating action determination unit 17 detects the decrease and increase patterns illustrated in FIG. 18 from the acceleration in the front-and-back direction acquired with the acceleration data acquisition unit 12. When front-and-back reciprocal patterns corresponding to the eating action are detected, as an example, the eating action determination unit 17 stores the decrease start time and the increase end time forming the pattern, or the duration period of the reciprocal movement from the decrease start time, for each of the front-and-back reciprocal patterns corresponding to the eating action, in an internal memory not illustrated.

The communication I/F unit 18 is an interface controlling communication with another apparatus, such as the server apparatus 100.

As an embodiment, a network interface card such as a LAN card can be adopted as the communication I/F unit 18. For example, the communication I/F unit 18 transmits the feature quantities calculated with the feature quantity calculator 16 for each of pieces of the window data, and the eating action information determined with the eating action determination unit 17, such as the decrease start time and the increase end time forming the pattern, to the server apparatus 100. The communication I/F unit 18 also receives designation of the type and the number of feature quantities to be calculated, the estimation result of the meal time, and the diagnosis result using it.

The functional units such as the heart rate data acquisition unit 11, the acceleration data acquisition unit 12, the exercise period determination unit 13, the noise heart rate removal unit 14, the window data preparation unit 15, the feature quantity calculator 16, and the eating action determination unit 17 can be mounted as follows. For example, the functional units can be achieved by causing a central processing unit (CPU) to develop and execute processing to perform the same functions as those of the heart rate data acquisition unit 11, the acceleration data acquisition unit 12, the exercise period determination unit 13, the noise heart rate removal unit 14, the window data preparation unit 15, the feature quantity calculator 16, and the eating action determination unit 17, on the memory. These functional units are not always executed with the central processing unit, but may be executed with a micro processing unit (MPU). In addition, the functional units described above may be achieved with a hard wired logic such as an application specific integrated circuit (ASIC), and a field programmable gate array (FPGA).

In addition, the main storage device used with the functional units may be a semiconductor memory element of various types, such as a random access memory (RAM) and a flash memory, as an example. The storage device referred to by the functional units is not always the main storage device, but may be an auxiliary storage device. In such a case, the auxiliary storage device may be a hard disk drive (HDD), an optical disk, or a solid state drive (SSD).

Configuration of Server Apparatus 100

The following is explanation of the functional configuration of the server apparatus 100 according to the present embodiment. As illustrated in FIG. 1, the server apparatus 100 includes a communication I/F unit 110, a feature quantity storage unit 120, a model preparation unit 130, a first determination unit 140, a second determination unit 150, and a service providing unit 160. The server apparatus 100 may also include functional units included in a known computer, such as an input/output device of various types, as well as the functional units illustrated in FIG. 1.

The communication I/F unit 110 is an interface controlling communication with another device, such as the sensor terminal 10.

As an embodiment, a network interface card such as a LAN card can be adopted as the communication I/F unit 110. For example, the communication I/F unit 110 receives feature quantities for each piece of window data, and the eating action information and the like from the sensor terminal 10. The communication I/F unit 110 also transmits designation of the type and the number of feature quantities to be calculated, the estimation result of the meal time, and the diagnosis result using it.

The feature quantity storage unit 120 is a storage unit storing feature quantities of heartbeat relating to the meal.

As an embodiment, the feature quantity storage unit 120 stores each set of feature quantities calculated from the common window data, in association with a correct class indicating whether the user had a meal in a section corresponding to the window data, as teacher data, to prepare a meal estimation model by machine learning. The feature quantity storage unit 120 is capable of storing a set of feature quantities of a desired type and the desired number among the fourteen feature quantities of the five types, for each piece of window data. As an example of the "class" described above, a class of either of "meal" and "non-meal" is stored in the feature quantity storage unit 120.

The model preparation unit 130 is a processor preparing a meal estimation model.

As an embodiment, this explanation illustrates the case where the model preparation unit 130 uses a support vector machine as an example of the machine learning algorithm. When such a support vector machine is used to perform classification of the feature quantity vectors observed in the sensor terminal 10, the output value of the meal estimation model is expressed with a weighted sum of Kernel functions with a support vector serving as argument, as indicated with the following Expression (1).

$$y = \sum_{i \subset I^+} \mathbf{c}_i K\!\left(\mathbf{x}, \mathbf{x}_i^{(\sup\ port)}\right) - \sum_{j \subset I^-} \mathbf{c}_j K\!\left(\mathbf{x}, \mathbf{x}_j^{(\sup\ port)}\right)$$

$$K(\mathbf{x}, \mathbf{x}') = \exp\left(-\frac{\|\mathbf{x} - \mathbf{x}'\|^2}{2\gamma^2}\right)$$

Expression (1)

The symbol "y" in Expression (1) described above indicates an output value of the meal estimation model. In addition, the symbol "x" indicates a feature quantity vector observed in the sensor terminal 10. The symbol "x(support)" indicates a feature quantity vector located in the vicinity of the identification boundary of the classification, that is, a support vector, among the feature quantity vectors stored in advance as the teacher data in the feature quantity storage unit 120. The symbol "$c_i$" indicates a weight applied to the support vector. The symbol "$\gamma$" indicates a function parameter. The symbol "$1^+$" in Expression (1) indicates an index set of the support vectors of the class "meal", and the symbol "$1^-$" indicates an index set of the support vectors of the class "non-meal". This explanation illustrates a support vector machine as an example of machine learning; however, desired algorithms such as boosting and neural network may be applied, other than the support vector machine.

On such assumption, the model preparation unit 130 applies the optimization algorithm in the support vector machine, to specify the support vector x(support) and a weight c thereof, and thereby prepare the meal estimation model.

FIG. 19 is a diagram illustrating an example of the identification boundary. For the sake of convenience of explanation, FIG. 19 illustrates a two-dimensional vector space in which two feature quantities among the feature quantities are extracted as explanation variables; however, the meaning thereof is the same also in a vector space of the number of dimensions corresponding to the number of feature quantities used for the meal estimation model. In FIG. 19, black circles indicate feature quantity vectors with the class "meal" among feature vectors of the teacher data, cross marks indicate feature quantity vectors with the class "non-meal", and feature quantity vectors positioned in the vicinity of the identification boundary are enclosed with circular solid lines.

As illustrated in FIG. 19, in the support vector machine, the feature quantity vectors in the vicinity of the identification boundary, that is, support vectors, are used, to learn the identification boundary with which the width of the margin of the identification boundary classifying the vectors into the class "meal" and the class "non-meal" is maximized. In this manner, the weight c applied to each support vector in Expression (1) described above is learned. The feature quantity vector observed in the sensor terminal 10 is substituted for the corresponding symbol in the meal estimation model acquired by such learning, to acquire an output value having a value from -1 to +1.

The first determination unit 140 is a processor determining whether the section corresponding to each piece of window data is "meal" or "non-meal" using the meal estimation model described above.

As an embodiment, the first determination unit 140 applies the feature quantity of the window data to the meal estimation model prepared with the model preparation unit 130, for each feature quantity of the window data received from the sensor terminal 10, to determine whether meal exists in the window data. For example, the first determination unit 140 substitutes each of feature quantity vectors observed in the sensor terminal 10 for the corresponding value in Expression (1) described above. In this manner, the output value of the meal estimation model taking a value from -1 to +1 is acquired. When the output value of the meal estimation model is equal to or higher than a predetermined threshold, for example, 0, the first determination unit 140 classifies the window data into the class "meal". By contrast, when the output value of the meal estimation model is less than a predetermined threshold, for example, 0, the first determination unit 140 classifies the window data into the class "non-meal". In this manner, determination is performed as to the existence of meal, for each piece of window data.

The second determination unit 150 is a processor further determining occurrence of a meal in the window data determined to be meal with the first determination unit 140, using the eating action information detected from the acceleration data.

As an embodiment, the second determination unit 150 determines whether at least a predetermined number of the eating action patterns are included in a predetermined section, that is, a section of up to 20 minutes from the meal start time, in the window data determined to be meal with the first determination unit 140. Specifically, when the user has a meal, it is rare that the number of times of the action of carrying food to the mouth is a small number such as one and two, and it is more likely that the action is repeated a certain number of times. For this reason, the second determination unit 150 does not directly determine the occurrence of meal even when the first determination unit 140 determines occurrence of meal. The second determination unit 150 determines that the window data corresponds to a meal when the action is repeated a certain number of times, for example, ten times. By contrast, when the predetermined number of the eating actions is not included in the predetermined section of the window data, the second determination unit 150 determines that the window data corresponds to non-meal.

Thereafter, the second determination unit 150 specifies the meal time, for example, the meal start time, from the window data determined to correspond to a meal. In the operation, the second determination unit 150 can specify the time resulting from subtracting the pre-meal section from the window data as the meal start time, or specify the time at which the pattern corresponding to the eating action is detected first in the predetermined section of the window data, as the meal start time. In addition, the second determination unit 150 does not always specify the meal start time alone, but may also specify the meal end time. For example, the second determination unit 150 may specify the fixed time after the meal start time, such as 15 minutes, 30 minutes, and one hour after the meal start time, as the meal end time, or may specify the time at which the pattern corresponding to the eating action is detected last in the predetermined section of the window data as the meal end time. At least one of the meal start time, the meal end time, and a combination thereof specified as described above is output to the service providing unit 160.

The service providing unit 160 is a processor providing the health care support services described above.

As an embodiment, the service providing unit 160 records the meal time, such as at least one of the meal start time, the meal end time, and the meal start time and the meal end time, generates and outputs a list of the meal time periods for a predetermined period, such as one week, from the meal times recorded in the past, performs analysis relating to the dietary habits or diet based on the meal time recorded in the past, and outputs various advices.

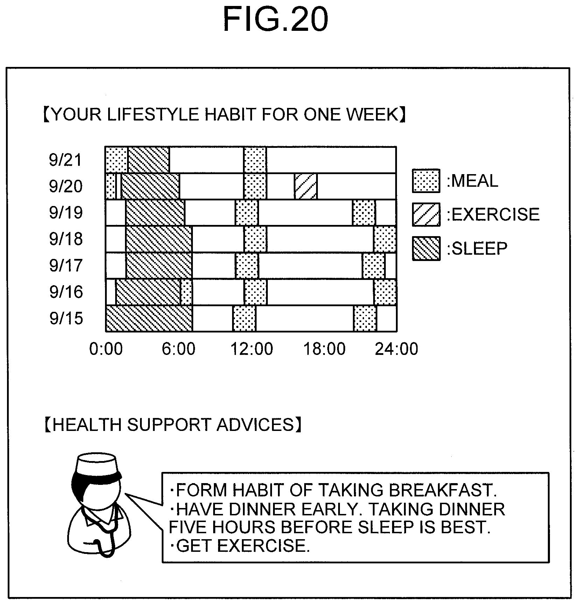

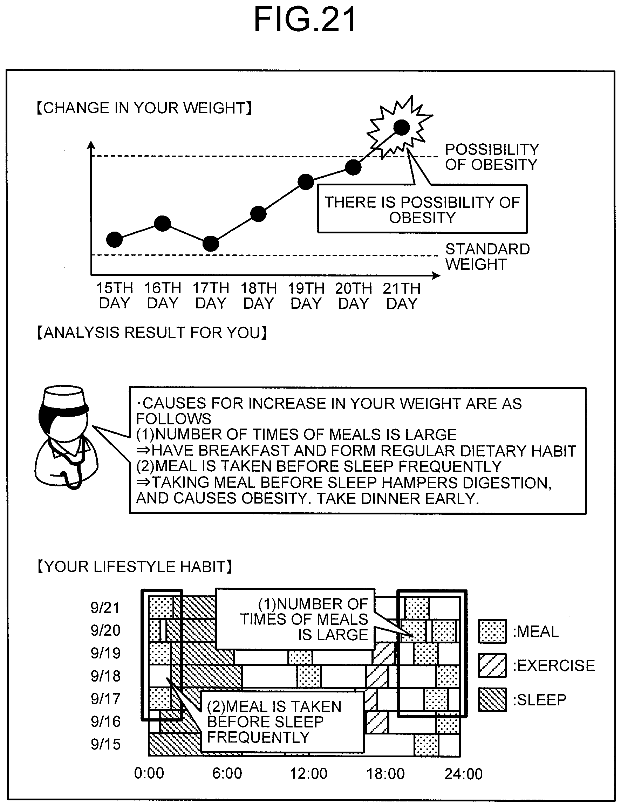

FIG. 20 and FIG. 21 are diagrams illustrating examples of the health care support services. Among them, FIG. 20 illustrates an output example in the case where lifestyle habit monitoring is performed, while FIG. 21 illustrates an output example in which lifestyle habit analysis for diet is performed. The display layouts illustrated in FIG. 20 and FIG. 21 are mere examples, and the layouts are not limited to the examples illustrated in FIG. 20 and FIG. 21, as a matter of course.

For example, analysis of lifestyle habits is enabled by linking the meal time described above with schedules of exercise and sleep. Specifically, as illustrated in FIG. 20, a time table relating to the lifestyle habits of the day for each day included in one week can be displayed. Such time table is capable of displaying attributes relating to lifestyle habits, such as the time periods in which the lifestyle habit is performed, for each of the meal, exercise, and sleep. In addition, as illustrated in FIG. 20, the time table is capable of displaying advices relating to lifestyle habits including the meals. For example, when analysis results are obtained regarding lifestyle habits such as taking no breakfast, taking a meal before sleep, and lack of exercise, the time table is capable of displaying advices such as "Form the habit of taking breakfast", "Have dinner early", and "Get exercise", as illustrated in FIG. 20.

In addition, analysis to promote diet is possible by further displaying information such as the weight, in addition to exercise and sleep, together with the meal time described above. Specifically, as illustrated in FIG. 21, the health care support service is capable of displaying a time table relating to lifestyle habits of the day for each day included in one week. In addition, as illustrated in FIG. 21, advices to promote diet can also be displayed. For example, when factors that excessive number of times of meals, and taking a meal before sleep are acquired as analysis results corresponding to the factors of fatness, the following display can be achieved. For example, as illustrated in FIG. 21, messages such as "the number of times of meals is large" and "a meal is taken before sleep frequently" can be displayed as causes for increase in weight. In addition, messages can be displayed as measures against it, such as "take breakfast and form a regular dietary habit", and "Taking a meal before sleep hampers digestion, and causes obesity. Take dinner early".

The functional units such as the model preparation unit 130, the first determination unit 140, the second determination unit 150, and the service providing unit 160 are mounted as follows. For example, the functional units can be achieved by causing the central processing unit or the like to develop and execute a process to perform the same functions as those of the model preparation unit 130, the first determination unit 140, the second determination unit 150, and the service providing unit 160 on a memory. The functional units are not necessarily executed with the central processing unit, but may be executed with the MPU. The functional units described above may also be achieved by hard wired logic.

As an example, various semiconductor memory elements such as a RAM and a flash memory may be adopted as the main storage devices used by the functional units, including the feature quantity storage unit 120 described above. The storage devices referred to by the functional units described above are not necessarily main storage devices, but may be auxiliary storage devices. In this case, an HDD, an optical disk, or an SSD can be adopted.

Specific Example

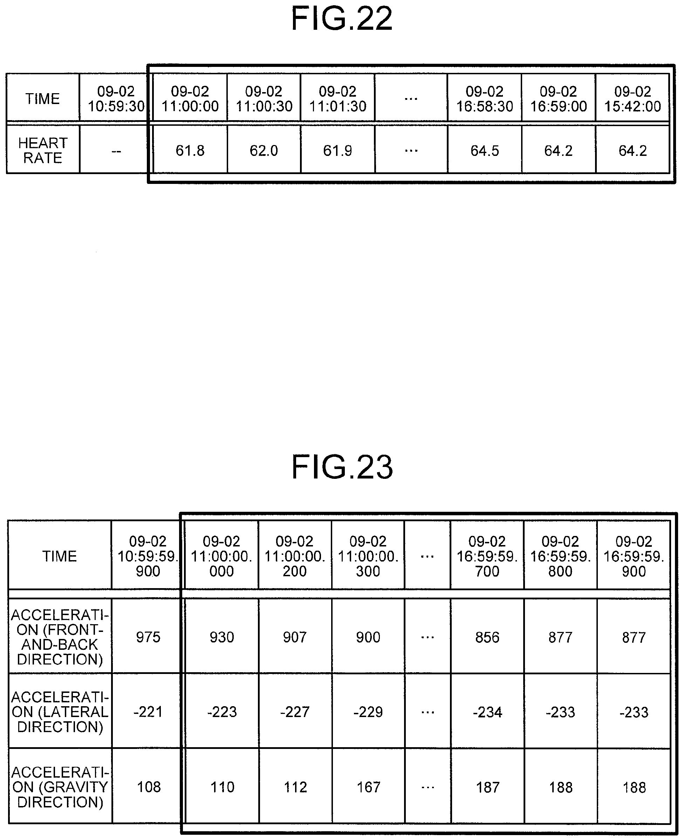

The following is explanation of a specific example relating to estimation of the meal time, with reference to FIG. 22 to FIG. 41. FIG. 22 is a diagram illustrating an example of heart rate data. FIG. 23 is a diagram illustrating an example of acceleration data. As an example, FIG. 22 illustrates heart rate data obtained by sensing the heart rate at 30-second cycles, and FIG. 23 illustrates acceleration data obtained by sensing the acceleration at 0.1-second cycles. In this example, suppose that the portions enclosed with solid lines in a rectangular shape, that is, the portions from 11:00:00 to 23:55:00 on September 2 are acquired as heart rate data and acceleration data, and FIG. 22 and FIG. 23 illustrate extracted data relating to part of the times among the data.

When the heart rate data illustrated in FIG. 22 and the acceleration data illustrated in FIG. 23 are acquired, the determination of the exercise period and the determination of the eating action described above are performed. For example, when determination of the exercise period is performed, the increase and decrease patterns illustrated in FIG. 14 are detected from the acceleration in the gravity direction among the accelerations included in the acceleration data illustrated in FIG. 23. Thereafter, when the intervals at which the pattern appears fall within the predetermined period, the section in which the pattern repeatedly appears in the predetermined period is determined to be exercise period. In addition, when determination of the eating action is performed, the patterns of front-and-back reciprocal movement corresponding to the eating actions, that is, the decrease and increase patterns illustrated in FIG. 18 are detected from the acceleration in the front-and-back direction among the accelerations included in the acceleration data illustrated in FIG. 23. Thereafter, it is determined that one eating action is performed, for each detection of a front-and-back reciprocal pattern corresponding to the eating action.

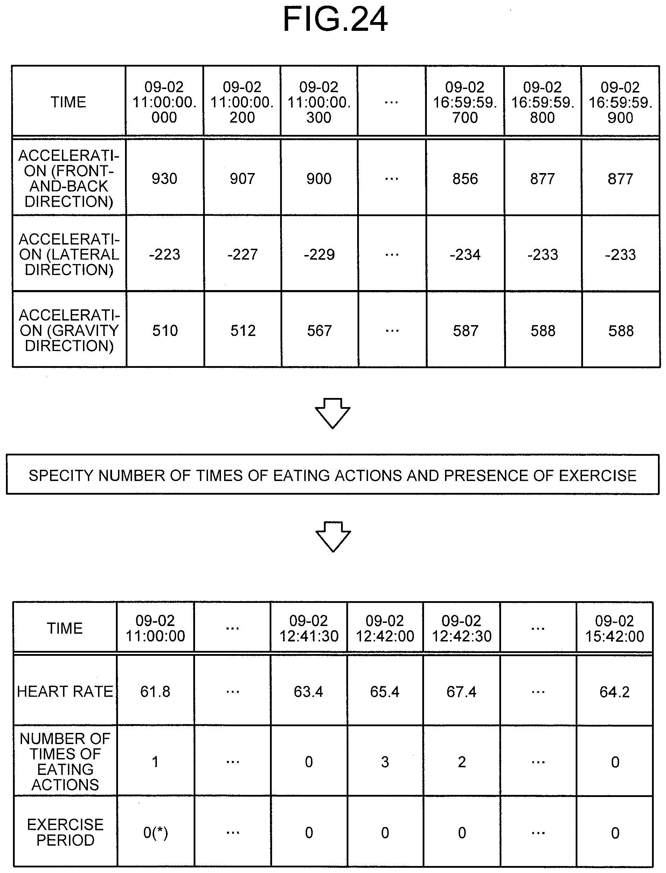

When the exercise periods and the eating actions are determined as described above, the exercise periods and the number of times of eating actions are managed on the internal memory of the sensor terminal 10, as illustrated in FIG. 24. FIG. 24 is a diagram illustrating an example of a table of correlation between the heart rate, the number of times of eating actions, and the exercise period. Although the acceleration data is sensed at 0.1-second cycles, FIG. 24 illustrates the number of times of eating actions and the exercise period for each 30 seconds, to correspond to 30 seconds serving as the sampling cycles of the heart rate data. For example, the record of the number of times of eating actions illustrated in FIG. 24 stores accumulation values of the number of times with which the front-and-back reciprocal pattern corresponding to an eating action is sensed for 30 minutes. In addition, in the record of the exercise period, "1" is set when the exercise period is included in 30 seconds, and "0" is set when no exercise period is included in 30 seconds. FIG. 24 illustrates the section from the time 11:00:00 to 11:00:30 on September 2, the section from the time 12:41:30 to 12:42:00 on the same date, the section from the time 12:42:00 to 12:42:30 on the same date, the section from the time 12:42:30 to 12:43:00 on the same date, and the section from the time 15:42:00 to 15:42:30 on the same date in an extracted manner; however, the values of the heart rate, the number of times of eating actions, and the exercise period are stored also for the other sections, as a matter of course. The following is explanation in which the column of the section is identified with the start time of the section, among the start time and the end time of the section.

FIG. 24 illustrates that no sections determined to be exercise period exist in the sections extracted in the correlation management table illustrated in FIG. 24. In addition, among the sections extracted in the correlation management table illustrated in FIG. 24, the number of times of eating actions is measured as 0 in the column of the time 12:41:30 and the column of the time 15:42:00, while one or more times of eating actions is measured in the other columns.

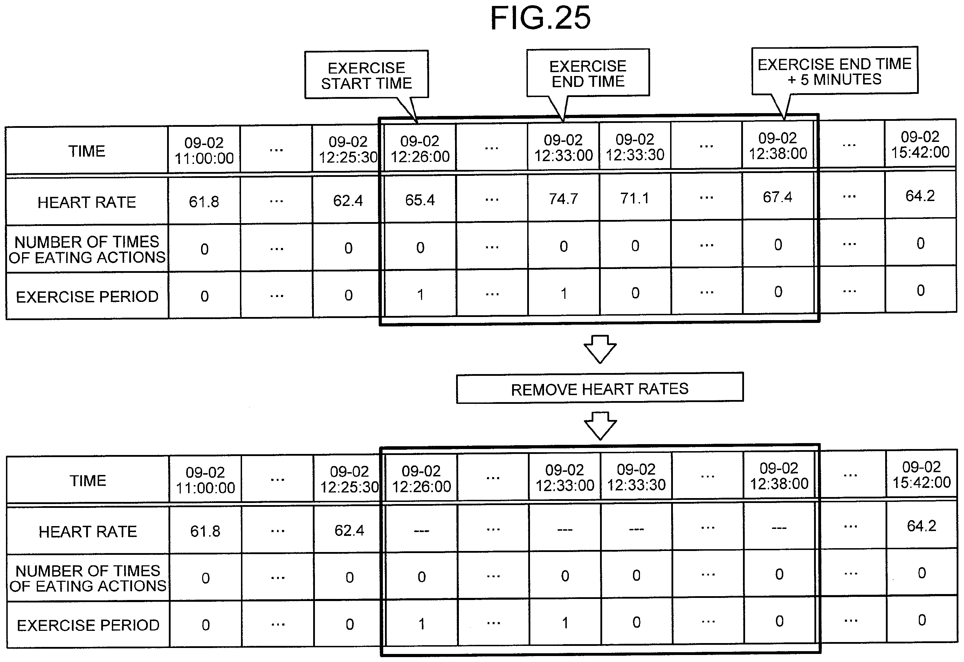

Under the circumstances in which the exercise periods are determined as described above, the section in which increase change and decrease change in heart rate is caused by exercise with high probability is removed as noise from the heart rate data illustrated in FIG. 22. FIG. 25 is a diagram illustrating an example of removal of the noise heart rate. The upper part of FIG. 25 illustrates a correlation management table before the noise heart rates are removed, while the lower part of FIG. 25 illustrates the correlation management table after the noise heart rates are removed. The correlation management tables illustrated in the upper part and the lower part of FIG. 25 are similar to that illustrated in FIG. 24; however, part of the extracted sections with the heart rate, the number of times of eating actions and the exercise time is different.

In the example of the correlation management table illustrated in the upper part of FIG. 25, the column of the time 12:26:00 corresponds to the exercise start time, while the column of the time 12:33:00 corresponds to the exercise end time. Specifically, the table indicates that the period from the time 12:26:00 to the time 12:33:00 is an exercise period. In this case, the period with the column of the time 12:26:00 serving as the start point, to the column of the time 12:38 obtained by adding a predetermined time, for example, five minutes, to the exercise end time is determined to be the removal period. In this case, as in the correlation management table illustrated in the lower part of FIG. 25, the heart rates of the part corresponding to the removal period are removed as noise heart rates, and changed to a blank state.

In the state where the noise heart rates are removed as described above, window data is prepared. In the case of the correlation management table illustrated in the lower part of FIG. 25, preparation of window data is started by matching the start time of the window with the time 11:00:00 serving as the start time of the heart rate data. For example, supposing that the window width is 210 minutes, the data string of the heart rate from the time 11:00:00 to 14:30:00 is extracted as window data. When the number to identify the window data is "1", the second window is set by shifting the window set as the window data "1" by a predetermined shift width, for example, five minutes. Specifically, window data "2" is prepared by matching the start time of the window with the time 11:05:00 of the heart rate data. Thereafter, the sliding processing is repeatedly performed until the end time of the window agrees with the time 23:55:00 serving as the end time of the heart rate data.

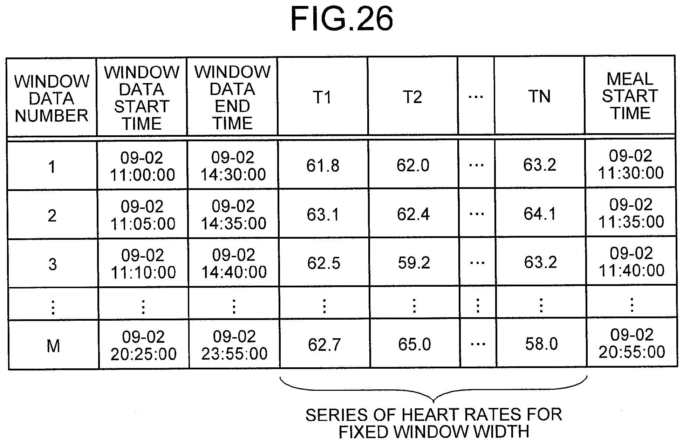

As a result, M pieces of window data illustrated in FIG. 26 are prepared. FIG. 26 is a diagram illustrating an example of window data extraction results. FIG. 26 illustrates M pieces of window data from window data "1" to window data "M". The items "T1" to "TN" illustrated in FIG. 26 indicate the data string of the heart rate data included in each piece of the window data. For example, in the case of the window data with the number "3" illustrated in FIG. 26, the table illustrates that the start time of the window data is the time 11:10:00 on September 2, and the end time is the time 14:40:00 on September 2. In addition, the table illustrates that the meal start time is the time 11:40:00 on September 2. The table also illustrates that the measured heart rates are 62.5, 59.2, ..., 63.2 for every 30 seconds from the time 11:10:00.

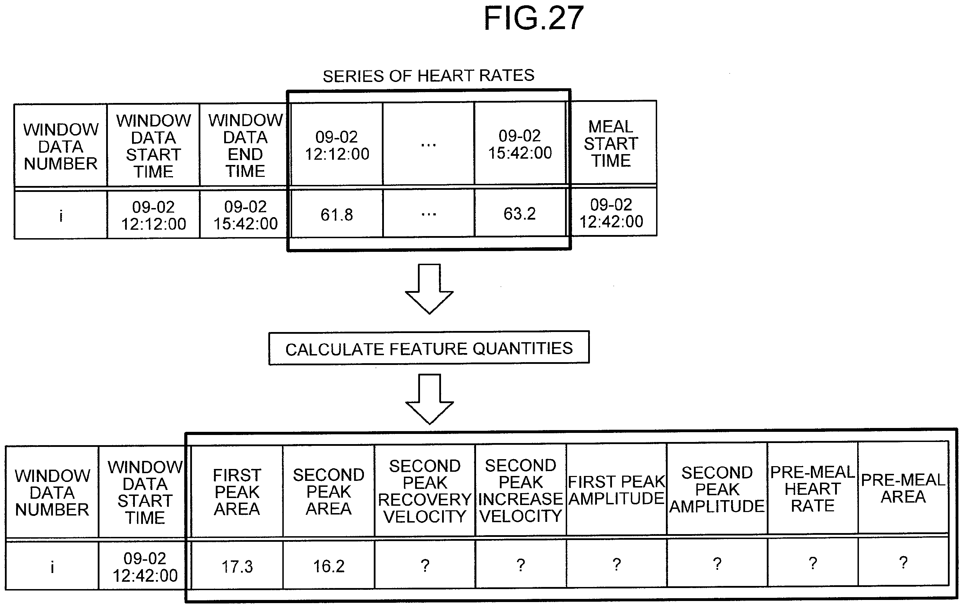

Thereafter, the feature quantities are calculated for each piece of window data. As an example, the explanation illustrates the case of calculating the feature quantities relating to the window data with the number "i" illustrated in FIG. 27. FIG. 27 is a diagram illustrating an example of window data. FIG. 27 illustrates the case of determining the first peak area, the second peak area, the second peak recovery velocity, the second peak increase velocity, the first peak amplitude, the second peak amplitude, the pre-meal heart rate, and the pre-meal area, as the feature quantities described above, from the heart rate series included in the ith window data. As illustrated in FIG. 27, the ith window data has the start time and the end time which are 12:12:00 and 15:42:00, respectively, and the meal start time of 12:42:00 that is 30 minutes after the start time of the window data.

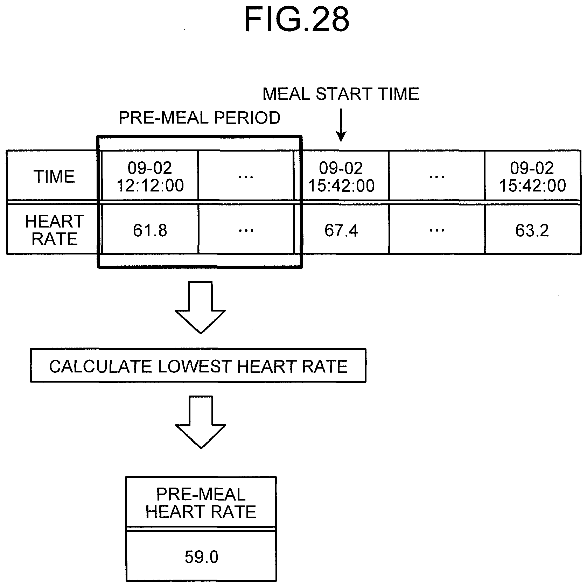

For example, in the case of calculating the feature quantities relating to the area, the baseline of the heart rate and the pre-meal heart rate are determined from the heart rate series included in the window data with the number "i". For example, among the heart rate series included in the window data with the number "i", the heart rate measured at the meal start time of the window data is determined to be "baseline". In addition, the heart rate determined to be "pre-meal heart rate" is the heart rate having the lowest value in the range from the start time to the meal start time of the window data among the heart rate series included in the window data with the number "i". FIG. 28 is a diagram illustrating an example of a method for determining the pre-meal heart rate. As illustrated in the upper part of FIG. 28, the pre-meal heart rate is determined from the heart rates included in the range from the start time of the window data to the meal start time, among the heart rate series included in the window data with the number "i". Specifically, as illustrated in the lower part of FIG. 28, the heart rate with the lowest value "59.0" is determined to be the pre-meal heart rate.

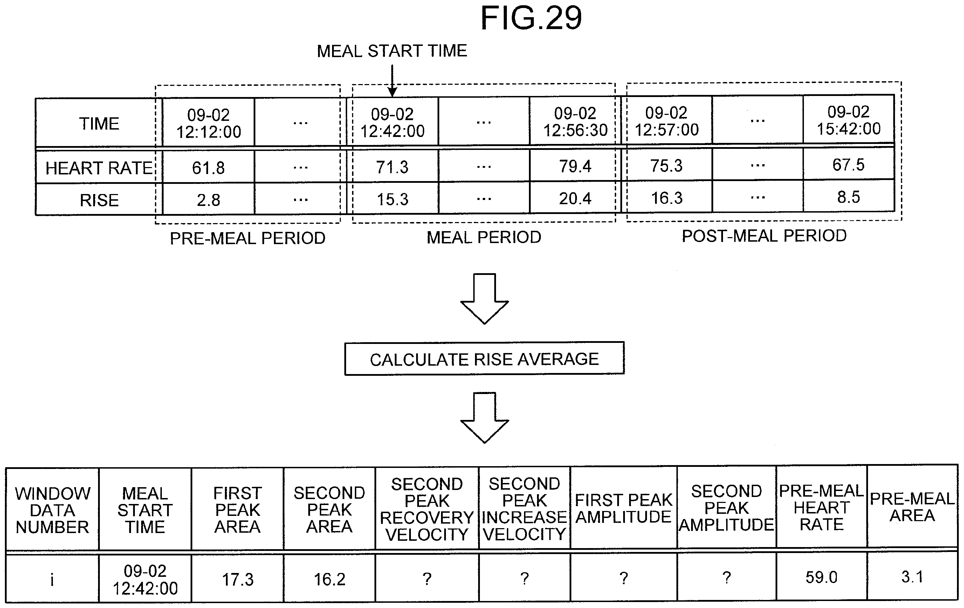

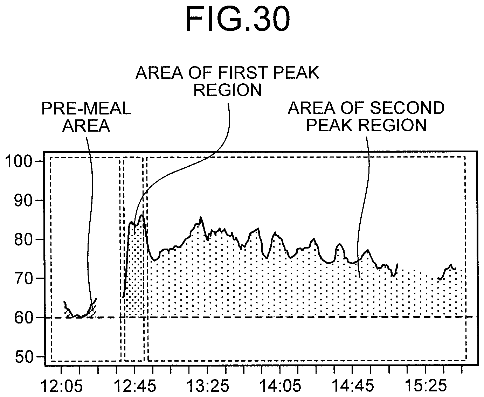

Thereafter, the heart rate series included in the window data with the number "i" is divided into three periods, that is, the pre-meal period, the meal period Ta1, and the post-meal period Ta2. FIG. 29 is a diagram illustrating an example of a process of calculating the feature quantities. FIG. 30 is a diagram illustrating an example of window data. The upper part of FIG. 29 illustrates the heart rate series and the rises included in the window data with the number "i", and the lower part of FIG. 29 illustrates calculation results of the pre-meal heart rate, the first peak area, the second peak area, and the pre-meal area, among the feature quantities relating to the window data with the number "i". FIG. 30 graphs the heart rate series included in the window data with the number "i". The vertical axis indicates the heart rate per unit time, and the horizontal axis indicates the elapsed time (time).

As illustrated in the upper part of FIG. 29, in the case of the window data with the number "i", the period from the start time "12:12:00" of the window data to the time, that is, the time "12:41:30", directly before the meal start time, is supposed to be the pre-meal period. In addition, the period from the meal start time "12:42:00" to a predetermined time after the meal start time, such as 15 minutes after the meal start time, that is, the time "12:56:30" is supposed to be the meal period. In addition, the period from the end time "12:57:00" of the meal time to the end time "15:42:00" of the window data is supposed to be the post-meal period. These three periods are graphed in FIG. 30.

In the graph, a difference is calculated for each time between the heart rate included in the pre-meal period and the pre-meal heart rate. In this manner, a rise in heart rate from the pre-meal heart rate is calculated for each time included in the pre-meal period. For example, in the case of the time "12:12:00", the rise "2.8" is calculated by subtracting the pre-meal heart rate "59.0" from the heart rate "61.8" measured at the time. After the rise in heart rate is calculated for each time included in the pre-meal period, a calculation is performed to average the rises of the heart rates of the respective times. In this manner, the pre-meal area illustrated in FIG. 30 is determined to be "3.1". As a result, as illustrated in the lower part of FIG. 29, the value "3.1" is stored as the value of the pre-meal area.

In addition, a difference is calculated for each time between the heart rate included in the meal period Ta1 and the pre-meal heart rate. In this manner, a rise in heart rate from the pre-meal heart rate is calculated for each time included in the meal period Ta1. For example, in the case of the time "12:42:00", the rise "12.3" is calculated by subtracting the pre-meal heart rate "59.0" from the heart rate "71.3" measured at the time. After the rise in heart rate is calculated for each time included in the meal period Ta1, a calculation is performed to average the rises of the heart rates of the respective times. In this manner, the first peak area illustrated in FIG. 30 is determined to be "17.3". As a result, as illustrated in the lower part of FIG. 29, the value "17.3" is stored as the value of the first peak area.

In addition, a difference is calculated for each time between the heart rate included in the post-meal period Ta2 and the pre-meal heart rate. In this manner, a rise in heart rate from the pre-meal heart rate is calculated for each time included in the post-meal period Ta2. For example, in the case of the time "12:57:00", the rise "16.3" is calculated by subtracting the pre-meal heart rate "59.0" from the heart rate "75.3" measured at the time. After the rise in heart rate is calculated for each time included in the post-meal period Ta2, a calculation is performed to average the rises of the heart rates of the respective times. In this manner, the second peak area illustrated in FIG. 30 is determined to be "16.2".

As a result, as illustrated in the lower part of FIG. 29, the value "16.2" is stored as the value of the second peak area.

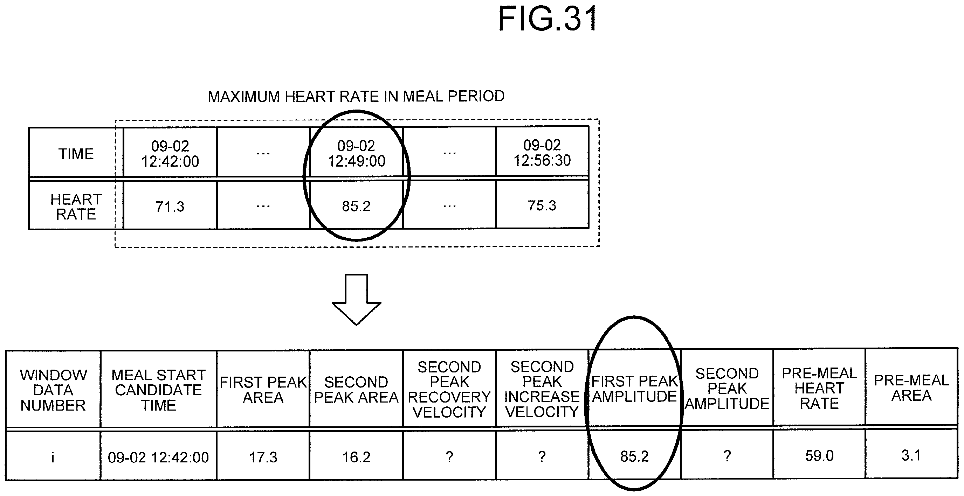

Thereafter, the amplitude of the first peak is determined. FIG. 31 is a diagram illustrating an example of a process of calculating feature quantities. The upper part of FIG. 31 illustrates data string of heart rates included in the meal period Ta1 in the window data with the number "i" in an extracted manner, and the lower part of FIG. 31 illustrates a calculation result of the amplitude of the first peak, in addition to the pre-meal heart rate, the first peak area, the second peak area, and the pre-meal area, in the feature quantities relating to the window data with the number "i".

As illustrated in FIG. 31, when the heart rate "85.2" measured at the time "12:49:00" among the data string of the heart rates included in the meal period Ta1 is the maximum heart rate P1, the maximum heart rate P1 "85.2" is extracted as the amplitude of the first peak. As a result, as illustrated in the lower part of FIG. 31, the value "85.2" is stored as the value of the amplitude of the first peak.

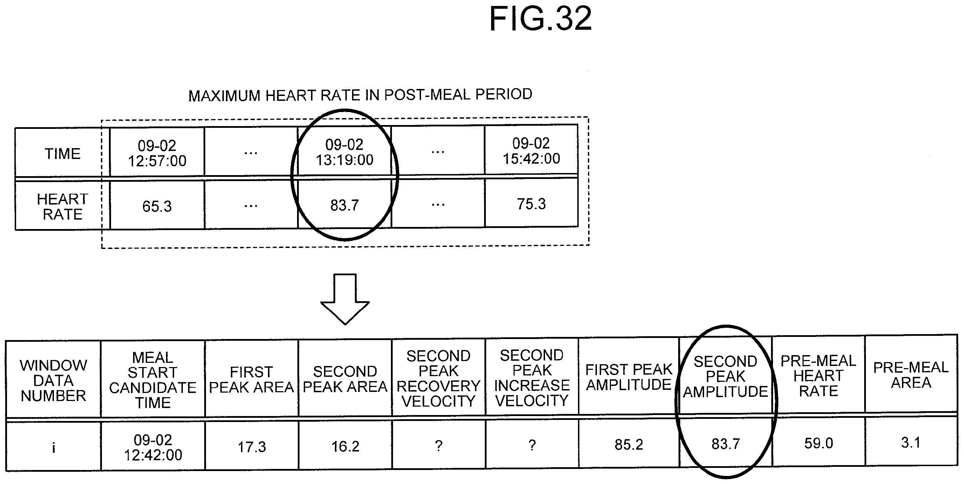

In addition, the amplitude of the second peak is determined. FIG. 32 is a diagram illustrating an example of a process of calculating feature quantities. The upper part of FIG. 32 illustrates data string of heart rates included in the post-meal period Ta2 in the window data with the number "i" in an extracted manner, and the lower part of FIG. 32 illustrates a calculation result of the amplitude of the second peak, in addition to the pre-meal heart rate, the first peak area, the second peak area, the pre-meal area, and the amplitude of the first peak, in the feature quantities relating to the window data with the number "i".

As illustrated in FIG. 32, when the heart rate "83.7" measured at the time "13:19:00" among the data string of the heart rates included in the post-meal period Ta2 is the maximum heart rate P2, the maximum heart rate P2 "83.7" is extracted as the amplitude of the second peak. As a result, as illustrated in the lower part of FIG. 32, the value "83.7" is stored as the value of the amplitude of the second peak.

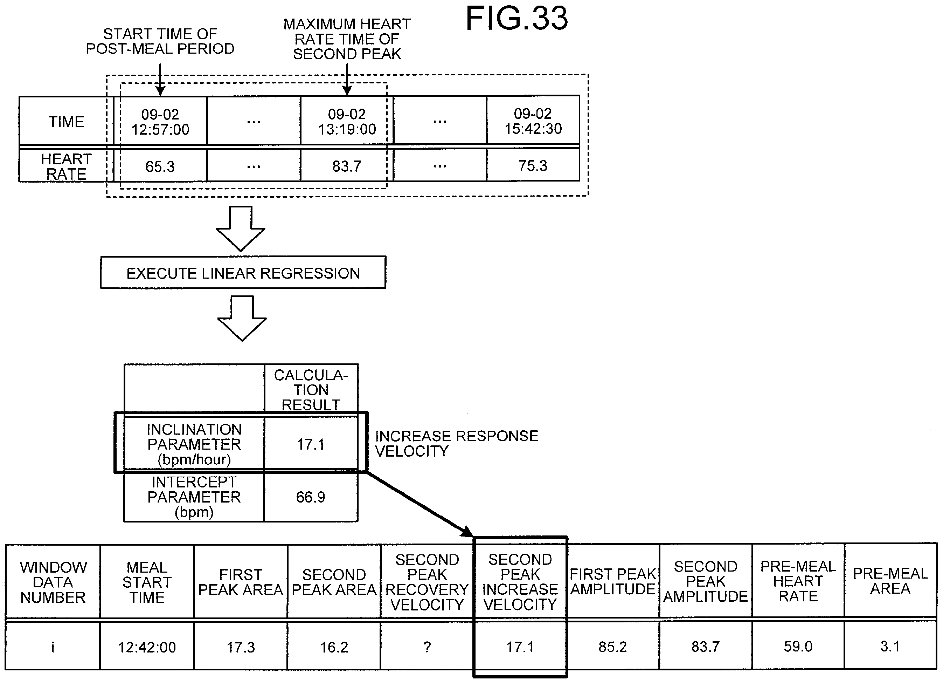

In addition, the increase velocity of the second peak is determined. FIG. 33 is a diagram illustrating an example of a process of calculating feature quantities. The uppermost part of FIG. 33 illustrates data string of heart rates included in the post-meal period Ta2 in the window data with the number "i" in an extracted manner, and the lowermost part of FIG. 33 illustrates a calculation result of the increase velocity of the second peak, in addition to the pre-meal heart rate, the first peak area, the second peak area, the pre-meal area, the amplitude of the first peak, and the amplitude of the second peak, in the feature quantities relating to the window data with the number "i".

When the increase velocity of the second peak is determined as described above, the following period is set as the approximation target period Ta2_P2. That is, the period set as the approximation target period Ta2_P2 includes the start time of the post-meal period Ta2, that is, the end time "12:57:00" of the meal period Ta1, as the start point, and the time "13:19:00" at which the maximum heart rate P2 of the second peak is measured, as the end point.

Thereafter, function approximation is performed using the data string of the heart rates included in the approximation target period Ta2_P2, to calculate the increase velocity of the second peak. For example, function approximation using a linear function such as "f (t) =$\alpha$t+$\beta$" is applicable. In approximation function using a linear function, function approximation suitable for the data string serving as the approximation target can be performed by determining a combination of the inclination parameter $\alpha$ and the intercept parameter $\beta$ to minimize the approximation error in each time included in the data string serving as the approximation target.

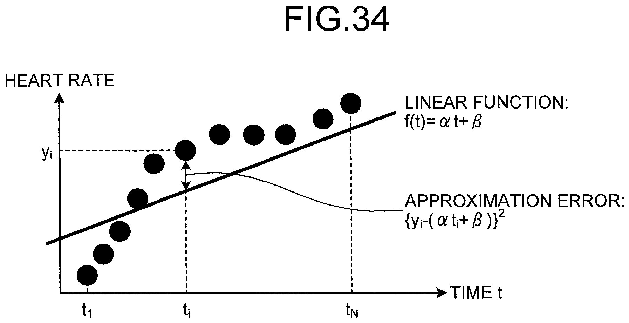

FIG. 34 is a diagram illustrating an example of the approximation error. FIG. 34 plots the data string of heart rates included in the approximation target period Ta2_P2 in the window data with the number "i" with black circles on the graph. The vertical axis of the graph indicates the heart rate per unit time, and the horizontal axis indicates the elapsed time (time). The time "t1" illustrated in FIG. 34 indicates, for example, the start time of the post-meal period Ta2, and the time "tN" indicates the time at which the maximum heart rate P2 was measured. FIG. 34 illustrates a linear function "f (t) =$\alpha$t+$\beta$" approximating the data string of the heart rates included in the approximation target period Ta2_P2, with a bold line.

As illustrated in FIG. 34, the approximation error between the heart rate yi at the time ti and the linear function "f (t) =$\alpha$t+$\beta$" can be expressed as a square of {yi-($\alpha$ti+$\beta$)}, as indicated in the following Expression (2). The approximation function suitable for the data string of the heart rates included in the approximation target period Ta2_P2 can be determined by determining a combination of the inclination parameter $\alpha$ and the intercept parameter $\beta$ minimizing the sum of squares of the approximation errors of heart rates at the respective times, that is, the sum of squares of {yi-($\alpha$ti+$\beta$)}. The symbol "$\alpha$" in the following Expression (2) denotes the inclination parameter of the approximation function, and the symbol "$\beta$" denotes the intercept parameter of the approximation function. The symbol "yi" indicates the ith heart rate, "ti" denotes the ith time, and "N" denotes the number of pieces of data serving as the approximation target.

$$E\left(\alpha, \beta\right) = \sum_{i=1}^{N} \left\{ y_i - \left(\alpha t_i + \beta\right) \right\}^2 \qquad\qquad \text{Expression (2)}$$

$\alpha$: inclination parameter
$\beta$: intercept parameter

$y_i$: heart rate of ith data

$t_i$: time of ith data

N: number of pieces of data in target range

For example, in the case of the data string of heart rates included in the approximation target period Ta2_P2 illustrated in the uppermost part of FIG. 33, the inclination parameter $\alpha$ is determined to be "17.1 (bpm/hour)", and the intercept parameter $\beta$ is determined to be "66.9 (bpm/hour)". As illustrated in the lowermost part in FIG. 33, the inclination parameter $\alpha$ "17.1" determined to be described above is stored as the increase velocity of the second peak.

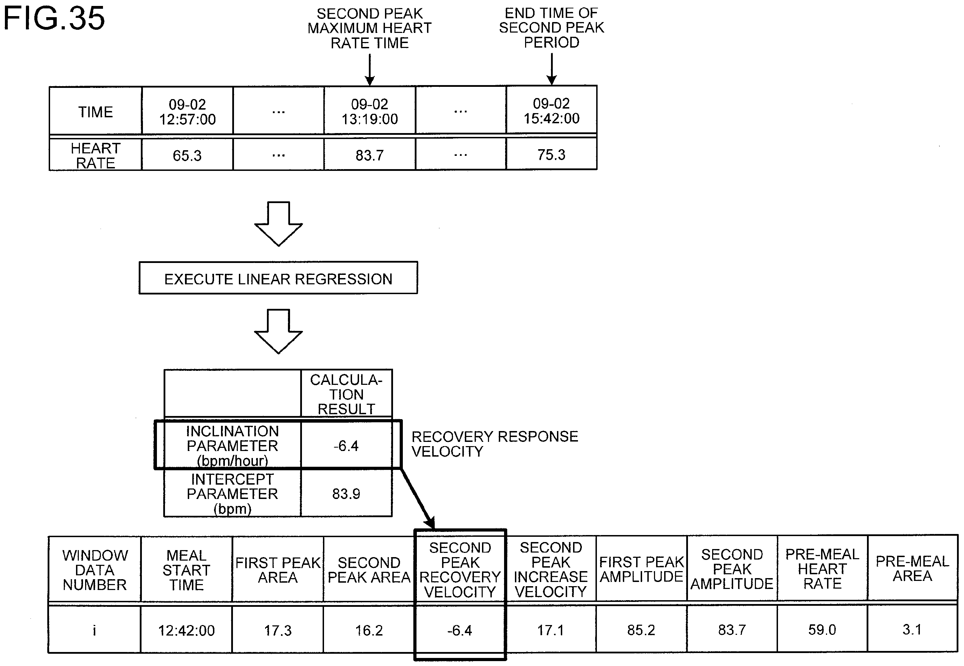

In addition, the recovery velocity of the second peak is determined. FIG. 35 is a diagram illustrating an example of a process of calculating the feature quantities. The uppermost part of FIG. 35 illustrates data string of heart rates included in the post-meal period Ta2 in the window data with the number "i" in an extracted manner, and the lowermost part of FIG. 35 illustrates a calculation result of the recovery velocity of the second peak, in addition to the pre-meal heart rate, the first peak area, the second peak area, the pre-meal area, the amplitude of the first peak, the amplitude of the second peak, and the increase velocity of the second peak, in the feature quantities relating to the window data with the number "i".

When the recovery velocity of the second peak is determined as described above, the following period is set as the approximation target period TP2_a2. That is, the period set as the approximation target period TP2_a2 includes the time at which the maximum heart rate P2 of the second peak is measured, that is, the time "13:19:00", as the start point, and the end time "15:42:00" of the post-meal period Ta2, as the end point.

Thereafter, function approximation is performed using the data string of the heart rates included in the approximation target period TP2_a2, to calculate the recovery velocity of the second peak, in the same manner as the case of calculating the increase velocity of the second peak described above. An approximation function suitable for the data string of heart rates included in the approximation target period TP2_a2 can be determined by determining a combination of the inclination parameter $\alpha$ and the intercept parameter $\beta$ minimizing the sum of squares of the approximation errors at the respective times, that is, the sum of squares of $\{y_i-(\alpha t_i+\beta)\}$.

As a result, in the case of the data string of heart rates included in the approximation target period TP2_a2 illustrated in the uppermost part of FIG. 35, the inclination parameter $\alpha$ is determined to be "-6.4 (bpm/hour)", and the intercept parameter $\beta$ is determined to be "83.9 (bpm/hour)". As illustrated in the lowermost part in FIG. 35, the inclination parameter $\alpha$ "-6.4" is stored as the recovery velocity of the second peak.

As described above, the first peak area, the second peak area, the second peak recovery velocity, the second peak increase velocity, the amplitude of the first peak, the amplitude of the second peak, the pre-meal heart rate, and the pre-meal area are determined, as the feature quantities, from the heart rate series included in the ith window data.

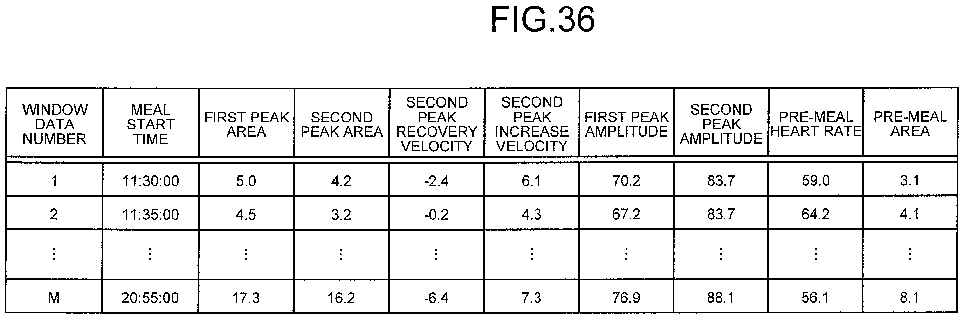

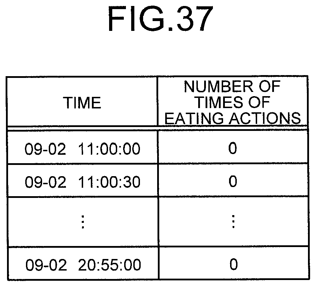

Thereafter, when feature quantities for all the pieces of window data are calculated, an eating action number-of-times list in which the numbers of eating actions for the respective times are listed is transmitted from the sensor terminal 10 to the server apparatus 100, together with an observation feature quantity list in which feature quantities of the respective pieces of window data are listed. FIG. 36 is a diagram illustrating an example of the observation feature quantity list, and FIG. 37 is a diagram illustrating an example of the eating action number-of-times list. As illustrated in FIG. 36, the observation feature quantity list includes the first peak area, the second peak area, the second peak recovery velocity, the second peak increase velocity, the amplitude of the first peak, the amplitude of the second peak, the pre-meal heart rate, and the pre-meal area that are calculated for each of pieces of window data from the number "1" to the number "M". The observation feature quantity list is transmitted from the sensor terminal 10 to the server apparatus 100. As illustrated in FIG. 37, the eating action number-of-times list includes the numbers of eating actions for the respective times from the start time of the heart rate data to the end time of the heart rate data. The eating action number-of-times list is transmitted from the sensor terminal 10 to the server apparatus 100.

The server apparatus 100 that has received the lists uses the feature quantity vector stored in advance as the teacher data in the feature quantity storage unit 120 and applies an optimization algorithm in the support vector machine, to specify the support vector $x^{(support)}$ and the weight c, and thereby prepare the meal estimation model.

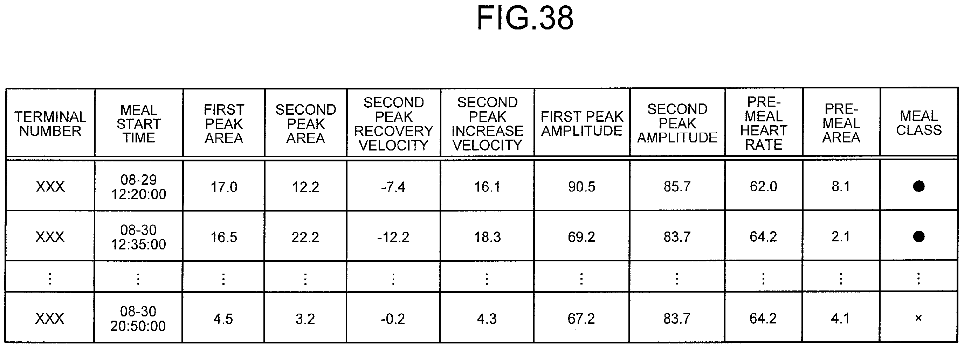

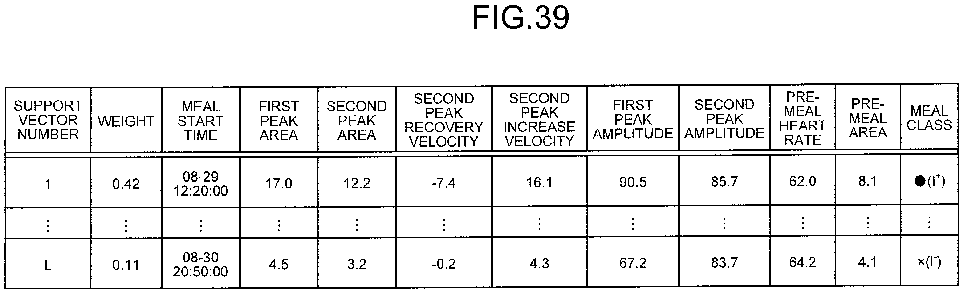

FIG. 38 is a diagram illustrating an example of teacher data. FIG. 39 is a diagram illustrating an example of the meal estimation model. As illustrated in FIG. 38, the teacher data is data with a correct answer, in which the meal class of the window data is associated with each piece of window data, together with the feature quantities such as the first peak area, the second peak area, the second peak recovery velocity, the second peak increase velocity, the amplitude of the first peak, the amplitude of the second peak, the pre-meal heart rate, and the pre-meal area. When the meal class illustrated in FIG. 38 is "● (black circle)", it indicates that the feature quantities of the window data are classified into "meal". By contrast, when the meal class is "x", it indicates that the feature quantities of the window data are classified into "non-meal". The teacher data illustrated in FIG. 38 indicates that the feature quantities of the window data with the meal start time "12:20:00 on August 29", and the feature quantities of the window data with the meal start time "12:35:00 on August 30" are classified into "meal". The teacher data also indicates that the feature quantities of the window data with the meal start time "20:50:00 on August 30" are classified into "non-meal". When learning is performed using the teacher data illustrated in FIG. 38, the meal estimation model illustrated in FIG. 39 is obtained. The meal estimation model illustrated in FIG. 39 indicates that L support vectors of "1" to "L" are located in the vicinity of the identification boundary of the classes. For example, the meal estimation model indicates that the support vector "1" is provided with the weight "0.42", and the support vector "L" is provided with the weight "0.11".

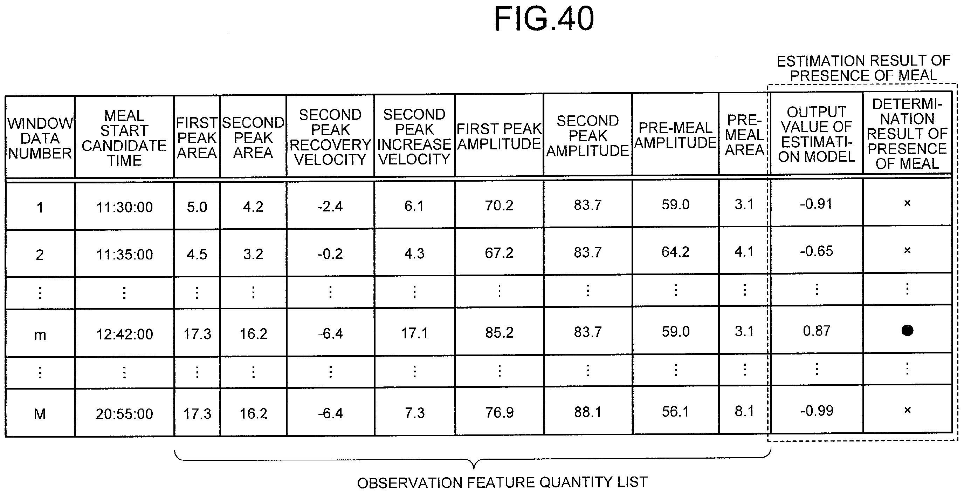

The feature quantity vectors included in the observation feature quantity list illustrated in FIG. 36 are substituted for corresponding values in the meal estimation model, to obtain the output results illustrated in FIG. 40. FIG. 40 is a diagram illustrating an example of estimation results of presence of meal. FIG. 40 is obtained by adding the output values of the meal estimation model and estimation results of presence of meal to the observation feature quantity list illustrated in FIG. 36. As can be seen, among the window data extracted in FIG. 40 in the window data with the number "1" to number "M", only the output value "0.87" of the window data with the number "m" is equal to or larger than 0, and the window data with the number "m" is classified into the class "meal".

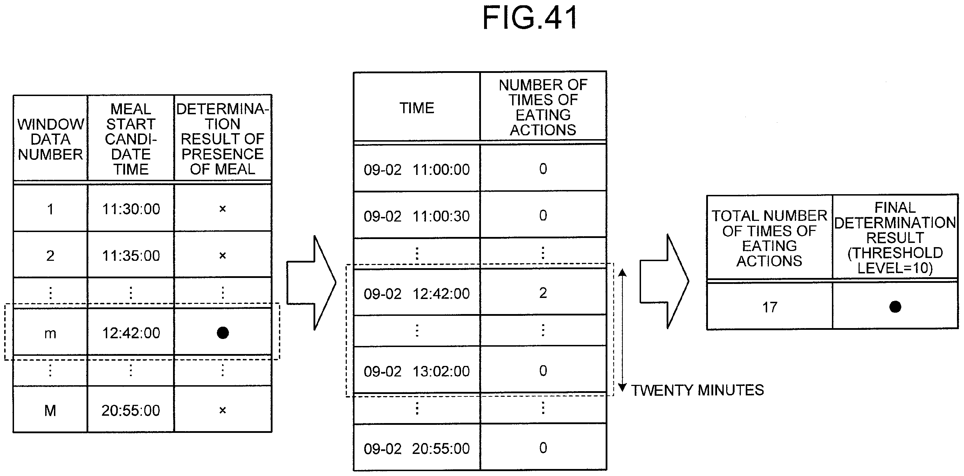

After determination of presence of meal is performed using the meal estimation model, it is further determined whether a meal was taken in the window data classified into "meal", using the eating action number-of-times list received from the sensor terminal 10. FIG. 41 is a diagram illustrating an example of meal determination using the eating action number-of-times list. The left part of FIG. 41 illustrates meal determination results, the middle part of FIG. 41 illustrates the eating action number-of-times list, and the right part of FIG. 41 illustrates the determination result. As illustrated in FIG. 41, in the meal determination using the meal estimation model, the window data with the number "m" is classified into the class "meal". For this reason, it is determined whether a predetermined number, for example, 10 or more eating actions are detected in the section of a predetermined period, for example, 20 minutes, from the meal start time of the window data with the number "m". In this example, because 17 eating actions are detected from the time "12:42:00" serving as the meal start time of the window data with the number "m" to the time "13:02:00", which is 20 minutes after the meal start time, the window data with the number "m" is determined to be "meal", as illustrated in the right part of FIG. 41.

Flow of Process

The following is explanation of the flow of the process of the health care support system according to the present embodiment. This explanation illustrates the whole flow executed by the sensor terminal 10 and the server apparatus 100, and thereafter illustrates the processing of calculating the feature quantities (1) to the feature quantities (5) executed by the sensor terminal 10.

(1) Whole Flow

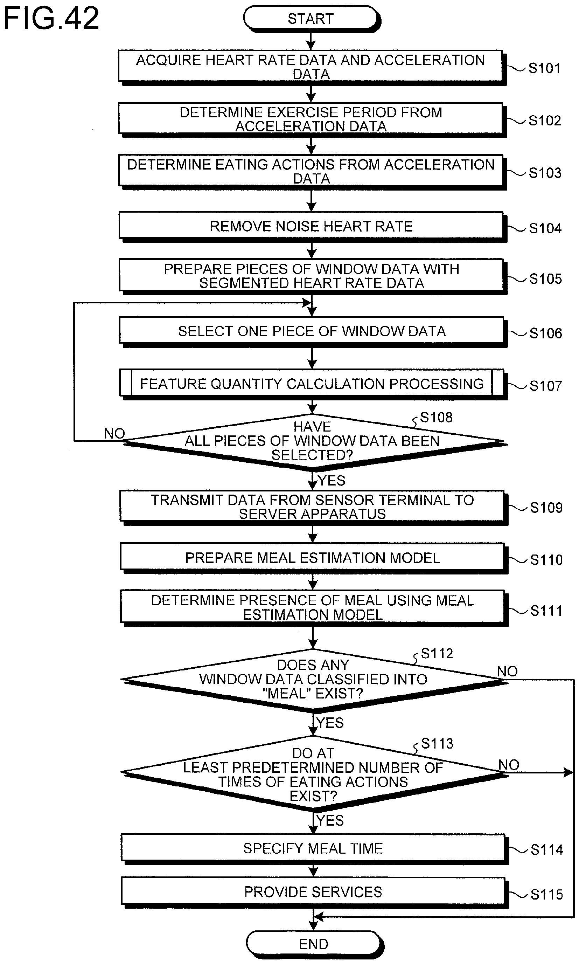

FIG. 42 is a flowchart illustrating a process of the whole flow according to the first embodiment. As an example, the processing is started when heart rate data and acceleration data are acquired for a predetermined time length, such as twelve hours and one day.

As illustrated in FIG. 42, when the heart rate data is acquired with the heart rate data acquisition unit 11 and the acceleration data is acquired with the acceleration data acquisition unit 12 (Step S101), the exercise period determination unit 13 executes the following processing. Specifically, the exercise period determination unit 13 determines a section in which intervals of the increase and decrease patterns corresponding to exercise repeatedly appear in the predetermined period, as the exercise period, using the acceleration in the gravity direction in the acceleration data acquired at Step S101 (Step S102).

Thereafter, the eating action determination unit 17 determines a front-and-back reciprocal pattern corresponding to the eating action using the acceleration in the front-and-back direction in the acceleration data acquired at Step S101 (Step S103).

Thereafter, the noise heart rate removal unit 14 removes the section corresponding to the removal period obtained by adding a fixed period to the exercise period determined at Step S102, from the heart rate data acquired at Step S101 (Step S104).

Thereafter, the window data preparation unit 15 extracts pieces of partial data in the window while shifting the window having a predetermined time length by a predetermined shift width until the end time of the window agrees with the end time of the heart rate data, from the heart rate data subjected to the removal at Step S104, to prepare pieces of window data with segmented heart rate data (Step S105).

Thereafter, the feature quantity calculator 16 selects one piece of window data from the window data prepared at Step S105 (Step S106). The feature quantity calculator 16 executes feature quantity calculation processing to calculate at least one of, or a combination of, the feature quantities (1) to (5), using the window data selected at Step S106 (Step S107).

Thereafter, the feature quantity calculator 16 repeatedly performs the processing at Step S106 and Step S107, until all the pieces of window data prepared at Step S105 are selected (No at Step S108).

When all the pieces of window data prepared at Step S105 are selected (Yes at Step S108), the communication I/F unit 18 of the sensor terminal 10 transmits the feature quantities calculated for each piece of window data at Step S107, and the eating action information determined at Step S103, such as the decrease start time and the increase end time forming the front-and-back reciprocal pattern corresponding to the eating action, to the server apparatus 100 (Step S109).

By contrast, the model preparation unit 130 of the server apparatus 100 specifies the support vector $x^{(support)}$ and the weight c thereof using the feature quantities with a correct answer stored as teacher data in the feature quantity storage unit 120, to prepare the meal estimation model (Step S110).

Thereafter, the first determination unit 140 determines presence of meal in the window data (Step S111), based on whether the output value output by substituting the feature quantity of the window data for the corresponding value in the meal estimation model prepared at Step S110 is equal to or higher than the predetermined threshold, for each feature quantity of the window data received from the sensor terminal 10. When the window data received from the sensor terminal 10 includes no window data classified into "meal" at Step S111 (No at Step S112), the processing is ended.

When the window data received from the sensor terminal 10 includes window data classified into "meal" at Step S111 (Yes at Step S112), the second determination unit 150 performs the following processing. Specifically, the second determination unit 150 determines whether at least a predetermined number of front-and-back reciprocal patterns corresponding to the eating actions are included in a predetermined section of the window data determined to be a meal at Step S111, that is, the section up to 20 minutes from the meal start time (Step S113). Also when no predetermined number or more of front-and-back reciprocal patterns corresponding to the eating actions are included in the section (No at Step S113), the processing is ended.

When at least a predetermined number of front-and-back reciprocal patterns corresponding to the eating actions are included in the section (Yes at Step S113), it is estimated that the supposed meal start time in the window data can be estimated to be valid with high probability. In this case, the second determination unit 150 specifies the meal time, that is, at least one of the meal start time, the meal end time, and a combination thereof, as the meal time from the window data (Step S114).

Thereafter, the service providing unit 160 records the meal time specified at Step S114, generates and outputs a list of the meal time periods for the predetermined period from the meal times recorded in the past, and performs analysis relating to the dietary habits or diet based on the meal times recorded in the past, and thereby outputs various advices, to provide the health care support services (Step S115), and ends the processing.

(2.1) Processing of Calculating Feature

Quantities (1)

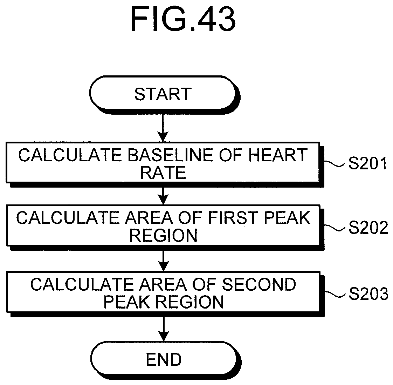

FIG. 43 is a flowchart illustrating a calculation processing process the feature quantities (1). The processing corresponds to Step S107. The processing may be executed in parallel with the processing of calculating the feature quantities (2) to feature quantities (5), or the processes may be executed in any order.

As illustrated in FIG. 43, as an example, the feature quantity calculator 16 derives the heart rate measured at the meal start time Ts included in the window data as baseline (Step S201). Thereafter, the feature quantity calculator 16 determines the area S1 of the first peak region A1 (Step S202), by summing or averaging the rises of the heart rate from the baseline BL derived at Step S201, in the meal period Ta1 including the meal start time Ts, as the start point, and the time at which the heart rate is recovered to the baseline BL through the first peak, as the end point.

The feature quantity calculator 16 determines the area S2 of the second peak region A2 (Step S203), by summing or averaging the rises of the heart rate from the baseline BL derived at Step S201, in the post-meal period Ta2 including the end point of the meal period Ta1 of the first peak region A1 or the time after the end point, as the start point, and the time at which the heart rate is recovered to the baseline BL through the second peak, as the end point. Thereafter, the feature quantity calculator 16 ends the processing. The value of the heart rate of each time may be a value obtained by moving and averaging the heart rates in the time periods before and after the time.

As described above, the area of the first peak region A1 and the area of the second peak region A2 are calculated as the feature quantity (1).

(2.2) Processing of Calculating Feature

Quantities (2)

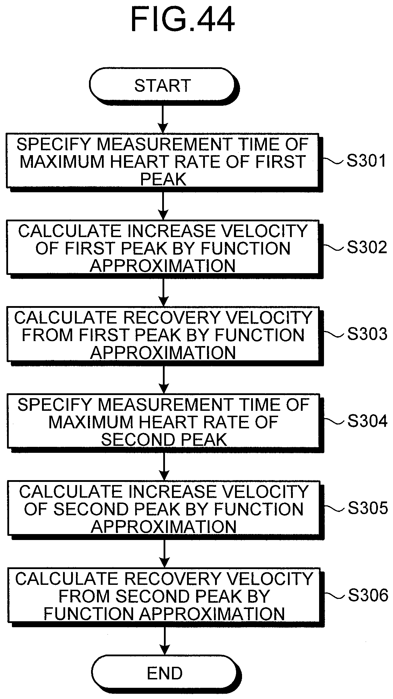

FIG. 44 is a flowchart illustrating a calculation processing process the feature quantities (2). The processing corresponds to Step S107. The processing may be executed in parallel with the processing of calculating the feature quantities (1), and the feature quantities (3) to feature quantities (5), or the processes may be executed in any order.

As illustrated in FIG. 44, the feature quantity calculator 16 specifies the time at which the maximum heart rate P1 of the first peak serving as the maximum heart rate is measured among the heart rates measured in the meal period Ta1 (Step S301).

Thereafter, the feature quantity calculator 16 sets the period from the meal start time Ts to the measurement time of the maximum heart rate P1 specified at Step S301, as the approximation target period Ts_P1, and performs function approximation on the data string of the heart rates included in the approximation target period Ts_P1, to calculate the increase velocity of the first peak (Step S302).

The feature quantity calculator 16 sets the period from the measurement time of the maximum heart rate P1 of the first peak to the time at which a fixed period is elapsed after the end of the meal, as the approximation target period Ta1_P1, and performs function approximation on the data string of the heart rates included in the approximation target period Ta1_P1, to calculate the recovery velocity of the first peak (Step S303).

The feature quantity calculator 16 specifies the time at which the maximum heart rate P2 of the second peak serving as the maximum heart rate is measured among the heart rates measured in the post-meal period Ta2 (Step S304).

Thereafter, the feature quantity calculator 16 sets the period from the start time of the post-meal period Ta2 to the measurement time of the maximum heart rate P2 specified at Step S304, as the approximation target period Ta2_P2, and performs function approximation on the data string of the heart rates included in the approximation target period Ta2_P2, to calculate the increase velocity of the second peak (Step S305).

Thereafter, the feature quantity calculator 16 sets the period from the measurement time of the maximum heart rate P2 of the second peak to the end time of the post-meal period Ta2, as the approximation target period TP2_a2, and performs function approximation on the data string of the heart rates included in the approximation target period TP2_a2, to calculate the recovery velocity of the second peak (Step S306). Thereafter, the feature quantity calculator 16 ends the processing.

As described above, the increase velocity of the heart rate until the heart rate reaches the first peak, the recovery velocity of the heart rate from the first peak, the increase velocity of the heart rate until the heart rate reaches the second peak, and the recovery velocity of the heart rate from the second peak are calculated as the feature quantities (2).

(2.3) Processing of Calculating Feature

Quantities (3)

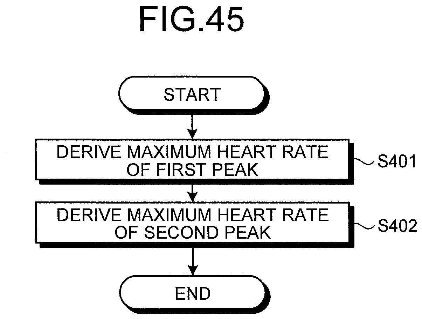

FIG. 45 is a flowchart illustrating a calculation processing process the feature quantities (3). The processing corresponds to Step S107. The processing may be executed in parallel with the processing of calculating the feature quantities (1) to the feature quantities (2), and the feature quantities (4) to feature quantities (5), or the processes may be executed in any order.

As illustrated in FIG. 45, the feature quantity calculator 16 derives the maximum heart rate P1 of the first peak serving as the maximum heart rate among the heart rates measured in the meal period Ta1 (Step S401). In addition, the feature quantity calculator 16 derives the maximum heart rate P2 of the second peak serving as the maximum heart rate among the heart rates measured in the post-meal period Ta2 (Step S402), and ends the processing.

As described above, the amplitude of the first peak, and the amplitude of the second peak are calculated as the feature quantities (3) described above.

(2.4) Processing of Calculating Feature

Quantities (4)

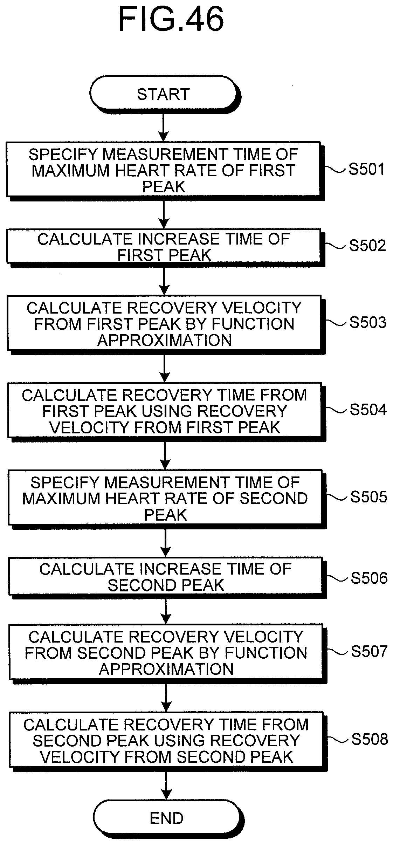

FIG. 46 is a flowchart illustrating a calculation processing process the feature quantities (4). The processing corresponds to Step S107. The processing may be executed in parallel with the processing of calculating the feature quantities (1) to the feature quantities (3) and the feature quantities (5), or the processes may be executed in any order.

As illustrated in FIG. 46, the feature quantity calculator 16 specifies the time at which the maximum heart rate P1 of the first peak serving as the maximum heart rate is measured among the heart rates measured in the meal period Ta1 (Step S501).

Thereafter, the feature quantity calculator 16 calculates the time from the start time of the meal period Ta1, that is, the meal start time Ts to the measurement time of the maximum heart rate P1 of the first peak derived at Step S501, as the time taken for increase of the first peak (Step S502).

Thereafter, the feature quantity calculator 16 sets the period from the measurement time of the maximum heart rate P1 of the first peak to the time at which a fixed period is elapsed after the end of the meal, as the approximation target period Ta1_P1, performs function approximation on the data string of the heart rates included in the approximation target period Ta1_P1, to calculate the recovery velocity of the first peak (Step S503).

Thereafter, the feature quantity calculator 16 calculates the recovery velocity of the first peak calculated at Step S503, that is, the elapsed time until the heart rate transitioning with the inclination of the approximation function reaches the heart rate of the baseline BL, as the recovery time from the first peak (Step S504).

In addition, the feature quantity calculator 16 specifies the time at which the maximum heart rate P2 of the second peak serving as the maximum heart rate is measured among the heart rates measured in the post-meal period Ta2 (Step S505).

Thereafter, the feature quantity calculator 16 calculates the time ranging from the start time of the meal period Ta1, that is, from the meal start time Ts, to the measurement time of the maximum heart rate P2 of the second peak specified at Step S505, as the time taken for increase of the second peak (Step S506).

Thereafter, the feature quantity calculator 16 sets the period from the measurement time of the maximum heart rate P2 of the second peak to the end time of the post-meal period Ta2, as the approximation target period TP2_a2, and performs function approximation on the data string of the heart rates included in the approximation target period TP2_a2, to calculate the recovery velocity of the second peak (Step S507).

Thereafter, the feature quantity calculator 16 calculates the recovery velocity of the second peak calculated at Step S507, that is, the elapsed time until the heart rate transitioning with the inclination of the approximation function reaches the heart rate of the baseline BL, as the recovery time from the second peak (Step S508), and ends the processing.

As described above, the increase time of the heart rate to the first peak, the recovery time of the heart rate from the first peak, the increase time of the heart rate to the second peak, and the recovery time of the heart rate from the second peak are calculated, as the feature quantities (4).

(2.5) Processing of Calculating Feature

Quantities (5)

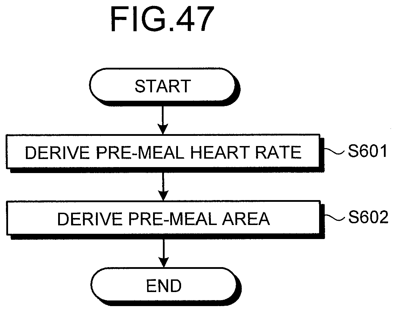

FIG. 47 is a flowchart illustrating a calculation processing process the feature quantities (5). The processing corresponds to Step S107. The processing may be executed in parallel with the processing of calculating the feature quantities (1) to the feature quantities (4), or the processes may be executed in any order.

As illustrated in FIG. 47, the feature quantity calculator 16 derives the heart rate having the lowest value among the heart rates measured in the pre-meal period ranging from the start time of the window data to the meal start time, as the pre-meal heart rate (Step S601).

Thereafter, the feature quantity calculator 16 calculates the pre-meal area (Step S602) by summing or averaging the rises of the heart rates from the pre-meal heart rate, in the pre-meal period ranging from the start time of the window data to the meal start time, and ends the processing.

As described above, the pre-meal heart rate and the pre-meal area are calculated as the feature quantities (5).

One Aspect of Effects

As described above, the health care support system 1 according to the present embodiment calculates feature quantities relating to the second peak appearing after the first peak appearing first after the start of the meal, and uses the feature quantities for estimation of the meal time, when the meal time is estimated from time-series data of the heart rate. Accordingly, the health care support system 1 according to the present embodiment suppresses decrease in accuracy of the meal determination.

In addition, the health care support system 1 according to the present embodiment calculates feature quantities relating to recovery change of the heart rate, as the feature quantities of the first peak and the second peak appearing after start of the meal, and uses the feature quantities for estimation of the meal time. With this structure, the health care support system 1 according to the present embodiment suppresses decrease in accuracy of the meal determination.

Experimental Example

The present embodiment is compared with the existing technique, with respect to accuracy of estimation of the meal time, to explain the advantageous effects of the present embodiment in comparison with the existing technique. For example, in the existing technique, the meal time is estimated using the rise of the heart rate of the first peak, while the meal time is estimated in the present embodiment using the fourteen feature quantities of five types described above.

On the premise described above, the existing technique and the present embodiment are compared with respect to the accuracy rate of classification into classes "meal" and "non-meal", that is, "the number of window data classified into the accurate class divided by the number of all the pieces of window data". As a result of experiments, it was proved that the average accuracy rate of the existing technique was "49.2%", while the average accuracy rate of the present embodiment was "98.4%". As described above, the present embodiment has an average accuracy rate substantially twice as high as that of the existing technique, and can be regarded as producing advantageous effects in comparison with the existing technique.

Second Embodiment

The embodiment relating to the disclosed apparatus has been described above; however, the present embodiment may be carried out in various different forms, in addition to the embodiment described above. The following is explanation of another embodiment included in the present invention.

Stand-alone

the first embodiment described above illustrates a client server system including the sensor terminal 10 and the server apparatus 100; however, the structure is not limited thereto. For example, a series of processes from acquisition of the heart rate data to estimation of the meal time may be executed with the sensor terminal 10, the server apparatus 100, or another computer in a stand-alone manner.

Application Example of System

the first embodiment described above illustrates the structure in which the server apparatus 100 is included in the health care support system 1; however, the server apparatus 1 is not always included. Specifically, when the sensor terminal 10 is mounted as a wearable gadget or the like, a smart phone or a tablet terminal connected with the wearable gadget by near-field communication or the like may be caused to perform various processes other than acquisition of the heart rate data, such as determination of the exercise time, determination of the eating action, calculation of the feature quantities, and estimation of the meal time.

Output of Information Other Than Meal Time

the first embodiment described above illustrates the case of outputting the meal start time, the meal end time, or a combination thereof; however, information other than the above may be output. For example, the server apparatus 100 is capable of outputting the end time of the post-meal time Ta2 and/or the recovery time from the second peak, as the time recommended as the time at which the next meal is to be eaten. This structure causes the user to take the next meal at the time when the digestive action is ended, and improves the quality of the dietary habits.

Application Example of Information Used Together with Heart Rate Data

The first embodiment described above illustrates the case of using acceleration data together with the heart rate for estimation of the meal time; however, the acceleration data is not necessarily used. As another example, information other than the acceleration data may be used.

Living Body Information

For example, living information can be used together with the heart rate for estimation of the meal time. Examples of living information that can be used together for estimation of the meal time include at least one of the body temperature, increase in skin temperature, increase in respiration rate, increase in blood pressure, increase in sweating quantity, increase in blood-sugar level, increase in weight, increase in girth of the chest or girth of the abdomen, increase in saliva quantity, sleepiness (after meal), and change in pigment quantity in the mouth or tongue, or a combination thereof.

Movement Information

In addition, movement information can be used together with the heart rate for estimation of the meal time. Examples of the movement information that can be used together for estimation of the meal time include at least one of movement of the whole face, movement of the mouth, movement of the head or the upper half of the body, movement of the arm in lifting up the food, movement of the stomach, the intestines, or the esophagus, and the forward-leaning posture, or a combination thereof.

Sound Information

Sound information can be used together with the heart rate for estimation of the meal time. Examples of the sound information that can be used together for estimation of the meal time include at least one of mastication sound, swallowing sound, slurping sound, and sound of tableware (the clink), or a combination thereof.

Text Information

Text information, such as a mail relating to the dietary action, or a keyword of an electric bulletin board or posted information can be used together with the heart rate for estimation of the meal time.

Surrounding Environmental Information

Surrounding environmental information can be used together with the heart rate for estimation of the meal time. Examples of the surrounding environmental information that can be used together for estimation of the meal time include at least one of position detection of the user in the place specific to the meal (store level, room level, or dining table level), image detection that food is included in an image, such as an image photographed by a wearable, an event of a smell of a meal, an event of a smell of exhalation, an event of a smell in the mouth, an event in which the object (such as a soy sauce bottle) is moved, an event in which the weight of the dish changes, an event in which the chair of the dining table is moved, and an event in which the ambient illuminance is higher than a threshold, or a combination thereof.

Pre-meal Action Pattern

In addition, a pre-meal action pattern can be used together with the heart rate for estimation of the meal time. Examples of the pre-meal action pattern that can be used together for estimation of the meal time include at least one of cooking habits (using gas, using tap water, using cooking tools), washing hands, shopping, increase in at least one of the number and the weight of food in the refrigerator, mastication sound, swallowing sound, slurping sound, and sound of tableware (the clink) with a predetermined time separated from the previous meal time, or a combination thereof.

Action Pattern During Meal

An action pattern during meal can be used together with the heart rate for estimation of the meal time. Examples of the action pattern during meal that can be used together for estimation of the meal time include at least one of a habit of often turning on the television, a habit of often taking jacket off, a habit of often spending time with others, habit of talking, a habit of taking a meal in a certain time period, a habit of taking a photograph during meal, and a condition that there is no action that would not occur simultaneously with a meal, such as a meeting and sleep, or a combination thereof.

Post-meal Action Pattern

A post-meal action pattern can be used together with the heart rate for estimation of the meal time. Examples of the post-meal action pattern that can be used together for estimation of the meal time include at least one of a habit of often going to the bathroom a predetermined time after the meal, a habit of often sleeping after the meal, a habit of often reducing the electronic money after the meal, and a habit of often loosening the belt after the meal, or a combination thereof.

Meal Estimation Program

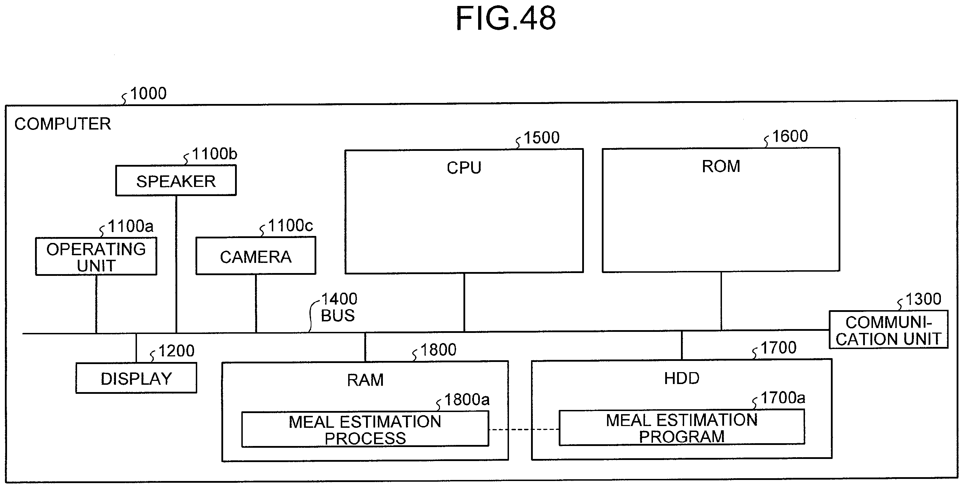

The various processes explained in the embodiments described above can be achieved by executing a program prepared in advance with a computer such as a personal computer and a workstation. The following is explanation of an example of a computer executing a meal estimation program having functions similar to those of the embodiments described above, with reference to FIG. 48.
FIG. 48 is a diagram illustrating a hardware configuration example of a computer executing the meal estimation program according to the first embodiment and the second embodiment. As illustrated in FIG. 48, a computer 1000 includes an operating unit 1100a, a speaker 1100b, a camera 1100c, a display 1200, and a communication unit 1300. The computer 1000 also includes a CPU 1500, a ROM 1600, a HDD 1700, a RAM 1800. The units 1100 to 1800 are connected through a bus 1400.
As illustrated in FIG. 48, the HDD 1700 stores therein a meal estimation program 1700a to perform functions similar to those of the heart rate data acquisition unit 11, the acceleration data acquisition unit 12, the exercise period determination unit 13, the noise heart rate removal unit 14, the window data preparation unit 15, the feature quantity calculator 16, the eating action determination unit 17, the model preparation unit 130, the first determination unit 140, the second determination unit 150, and the service providing unit 160 illustrated in the first embodiment described above. The meal estimation program 1700a may be integrated or separated, in the same manner as each of the constituent elements of the functional units illustrated in FIG. 1. Specifically, the HDD 1700 does not always store therein all the pieces of data illustrated in the first embodiment described above; and it suffices that data used for processing are stored in the HDD 1700.

Under such circumstances, the CPU 1500 reads the meal estimation program 1700a from the HDD 1700, and develops the meal estimation program 1700a onto the RAM 1800. As a result, the meal estimation program 1700a functions as a meal estimation process 1800a, as illustrated in FIG. 48. The meal estimation process 1800a develops various pieces of data read from the HDD 1700 onto a region assigned to the meal estimation process 1800a among a storage region included in the RAM 1800, and executes various types of processing using the developed data. Examples of the processing executed with the meal estimation process 1800a include the processes illustrated in FIG. 42 to FIG. 47. In the CPU 1500, not all the processors illustrated in the first embodiment necessarily operate; and it suffices that processors corresponding to the processing to be executed are virtually performed.

The meal estimation program 1700a is not always stored in the HDD 1700 or the ROM 1600 first. For example, each program is stored in a flexible disk inserted into the computer 1000, that is, a "portable physical medium" such as a FD, a CD-ROM, a DVD disk, a magneto-optical disk, and an IC card. Thereafter, the computer 1000 may acquire each program from the portable physical medium and execute the program. Each program may be stored in another computer or a server apparatus connected with the computer 1000 through a public line, the Internet, a LAN, or a WAN, and the computer 1000 may acquire each program from them and execute the program.

[Explanation of Reference]

| 1 | HEALTH CARE SUPPORT SYSTEM |
| 5 | NETWORK |
| 10 | SENSOR TERMINAL |
| 11 | HEART RATE DATA ACQUISITION UNIT |
| 12 | ACCELERATION DATA ACQUISITION UNIT |
| 13 | EXERCISE PERIOD DETERMINATION UNIT |
| 14 | NOISE HEART RATE REMOVAL UNIT |
| 15 | WINDOW DATA PREPARATION UNIT |
| 16 | FEATURE QUANTITY CALCULATOR |
| 17 | EATING ACTION DETERMINATION UNIT |
| 18 | COMMUNICATION I/F UNIT |
| 100 | SERVER APPARATUS |
| 110 | COMMUNICATION I/F UNIT |
| 120 | FEATURE QUANTITY STORAGE UNIT |
| 130 | MODEL PREPARATION UNIT |
| 140 | FIRST DETERMINATION UNIT |
| 150 | SECOND DETERMINATION UNIT |
| 160 | SERVICE PROVIDING UNIT |

**Claims**

1. A meal estimation program that causes a computer to execute a process comprising:

   acquiring time-series data of heart rate;
   calculating a feature quantity relating to a second peak appearing after a first peak in which a peak of the heart rate appears first after start of a meal, for each partial data included in the time-series data of the heart rate;
   determining presence of a meal in the partial data using the feature quantity relating to the second peak calculated for each of the partial data; and
   estimating a meal time from the partial data determined to include a meal.

2. The meal estimation program according to claim 1, wherein the calculating includes calculating an area formed of a partial waveform of the heart rate around the second peak, as the feature quantity relating to the second peak.

3. The meal estimation program according to claim 1, wherein the calculating includes calculating an increase velocity of the heart rate to the second peak and a recovery velocity of the heart rate from the second peak, as the feature quantity relating to the second peak.

4. The meal estimation program according to claim 1, wherein the calculating includes calculating an increase time of the heart rate to the second peak and a recovery time of the heart rate from the second peak, as the feature quantity relating to the second peak.

5. The meal estimation program according to claim 1,
wherein the calculating includes further calculating a feature quantity relating to the first peak.

6. The meal estimation program according to claim 1, the program that causes the computer to further execute a process including:

   acquiring time-series data of acceleration; and
   determining change in acceleration corresponding to an eating action of the meal from the time-series data of the acceleration, wherein
   the determining includes further determining presence of a meal in the partial data using the change in acceleration corresponding to an eating action of the meal.

7. The meal estimation program according to claim 1, the program that causes the computer to further execute a process including:

   acquiring time-series data of acceleration;
   determining change in acceleration corresponding to exercise from the time-series data of the acceleration; and
   removing a section of the change in acceleration corresponding to the exercise from the time-series data of the heart rate, or performing interpolation after the removing.

8. A meal estimation program that causes a computer to execute a process comprising:

   acquiring time-series data of heart rate;
   calculating a feature quantity relating to a recovery change from a first peak in which a peak of the heart rate appears first after start of a meal, and a feature quantity relating to recovery change from a second peak appearing after the first peak, for each partial data included in the time-series data of the heart rate;
   determining presence of a meal in the partial data using the feature quantities relating to the recovery changes of the first peak and the second peak calculated for each of the partial data; and
   estimating a meal time from the partial data determined to include a meal.

9. A meal estimation method comprising performing, with a computer, a process of:

   acquiring time-series data of heart rate;
   calculating a feature quantity relating to a second peak appearing after a first peak in which a peak of the heart rate appears first after start of a meal, for each partial data included in the time-series data of the heart rate;
   determining presence of a meal in the partial data using the feature quantity relating to the second peak calculated for each of the partial data; and
   estimating a meal time from the partial data determined to include a meal.

10. The meal estimation method according to claim 9, wherein the calculating includes calculating an area formed of a partial waveform of the heart rate around the second peak, as the feature quantity relating to the second peak.

11. The meal estimation method according to claim 9, wherein the calculating includes calculating an increase velocity of the heart rate to the second peak and a recovery velocity of the heart rate from the second peak, as the feature quantity relating to the second peak.

12. The meal estimation method according to claim 9, wherein the calculating includes calculating an increase time of the heart rate to the second peak and a recovery time of the heart rate from the second peak, as the feature quantity relating to the second peak.

13. A meal estimation apparatus comprising:

an acquisition unit configured to acquire time-series data of heart rate;

a calculator configured to calculate a feature quantity relating to a second peak appearing after a first peak in which a peak of the heart rate appears first after start of a meal, for each partial data included in the time-series data of the heart rate;

a determination unit configured to determine presence of a meal in the partial data using the feature quantity relating to the second peak calculated for each of the partial data; and

an estimation unit configured to estimate a meal time from the partial data determined to include a meal.

# FIG.1

1

**SENSOR TERMINAL** (10)

| | |
|---|---|
| HEART RATE DATA ACQUISITION UNIT (11) | ACCELERATION DATA ACQUISITION UNIT (12) |
| NOISE HEART RATE REMOVAL UNIT (14) | EXERCISE PERIOD DETERMINATION UNIT (13) |
| WINDOW DATA PREPARATION UNIT (15) | |
| FEATURE QUANTITY CALCULATOR (16) | EATING ACTION DETERMINATION UNIT (17) |
| | COMMUNICATION I/F UNIT (18) |

5

**SERVER APPARATUS** (100)

| | |
|---|---|
| SERVICE PROVIDING UNIT (160) | |
| SECOND DETERMINATION UNIT (150) | |
| FIRST DETERMINATION UNIT (140) | MODEL PREPARATION UNIT (130) |
| | FEATURE QUANTITY STORAGE UNIT (120) |
| COMMUNICATION I/F UNIT (110) | |

# FIG.2

# FIG.3

# FIG.4

(TIME AT WHICH HEART RATE
REACHES HIGHEST VALUE)

Ts

(2)

HEART RATE

(2)

(2)

(2)

(TIME AT WHICH HEART
RATE IS RECOVERED TO
HEART RATE BEFORE
MEAL)

TIME

12:00    14:00    16:00

(4)

t1
t2
t3
t4

# FIG.5

MEAL
PERIOD
(Ta1)

POST-MEAL
PERIOD
(Ta2)

HEART
RATE

A2

A1

BL

Ts

S1    S2

TIME

# FIG.6

MAXIMUM
HEART RATE
(P1)

APPROXIMATION
TARGET PERIOD
(Ts_P1)

APPROXI-
MATION
FUNCTION

INCLINATION OF
FUNCTION
CORRESPONDS TO
"INCREASE
VELOCITY"

MEAL
PERIOD
(Ta1)

# FIG.7

MAXIMUM
HEART RATE
(P1)

5
MINUTES

APPROXI-
MATION
TARGET
PERIOD
(Ta1_P1)

APPROXI-
MATION
FUNCTION

MEAL
PERIOD
(Ta1)

INCLINATION OF FUNCTION
CORRESPONDS TO
"RECOVERY VELOCITY"

# FIG.8

POST-MEAL
PERIOD(Ta2)

APPROXIMATION
FUNCTION

MAXIMUM HEART
RATE (P2)

APPROXIMATION
TARGET PERIOD
(Ta2_P2)

INCLINATION OF FUNCTION CORRESPONDS
TO "INCREASE VELOCITY"

(HEART RATE axis: 50, 60, 70, 80, 90, 100)
(TIME axis: 11:00 12:00 13:00 14:00 15:00 16:00 17:00 TIME)

# FIG.9

POST-MEAL
PERIOD(Ta2)

MAXIMUM HEART
RATE(P2)

APPROXI-
MATION
FUNCTION

APPROXIMATION TARGET
PERIOD(TP2_a2)

INCLINATION OF
FUNCTION CORRESPONDS
TO "RECOVERY VELOCITY"

(HEART RATE axis: 50, 60, 70, 80, 90, 100)
(TIME axis: 11:00 12:00 13:00 14:00 15:00 16:00 17:00 TIME)

# FIG.10

MEAL PERIOD
(Ta1)

MEAL PERIOD
(Ta2)

MAXIMUM
VALUE

HEART RATE

TIME

AMPLITUDE OF
FIRST PEAK(P1)

AMPLITUDE OF
SECOND PEAK(P2)

# FIG.11

MEAL PERIOD(Ta1)

APPROXI-
MATION
FUNCTION
FOR
RECOVERY
RESPONSE
OF FIRST
PEAK

MAXIMUM
HEART RATE
(P1)

HEART RATE

100
90
80
70
60
50

12:35  12:40  12:45  12:50  12:55  13:00  TIME

BASELINE
HEART
RATE(BL)
(Ts)

INCREASE
TIME

RECOVERY
TIME

TIME AT
WHICH HEART
RATE IS
RECOVERED
TO BASELINE
HEART RATE

35

# FIG.12

MEAL PERIOD(Ta1)

POST-MEAL PERIOD(Ta2)

APPROXIMATION FUNCTION FOR RECOVERY RESPONSE OF SECOND PEAK

MAXIMUM HEART RATE(P2)

HEART RATE

100
90
80
70
60
50

11:00  12:00  13:00  14:00  15:00  16:00  17:00  TIME

BASELINE HEART RATE(BL)

(Ts)

INCREASE TIME

RECOVERY TIME

TIME AT WHICH HEART RATE IS RECOVERED TO BASELINE HEART RATE

# FIG.13

60 MINUTES

FIRST PEAK

SECOND PEAK

HEART RATE

BL

PRE-MEAL AREA

PRE-MEAL HEART RATE

TIME

14:00      16:00

MEAL START TIME

# FIG.14

CHEST ACCELERATION (GRAVITY ACCELERATION) DURING WALKING

15000

10000

5000

RAPID ASCENT/DESCENT PATTERN APPEARS IN ACCELERATION IN GRAVITY DIRECTION DURING EXERCISE

TIME

# FIG.15

(1)

INCREASE IN HEART RATE
CAUSED BY EXERCISE

HEART
RATE

TIME

(2)

Px

ERRONEOUS FUNCTION
APPROXIMATION AND ERRONEOUS
FEATURE EXTRACTION ARE
PERFORMED DUE TO INCREASE IN
HEART RATE CAUSED BY EXERCISE

CHANGE IN HEART RATE
PRIMARILY ATTRIBUTED TO MEAL

(3)

REMOVE HEART RATE IN
EXERCISE PERIOD

# FIG.16

(1) 【DETECTION OF RAPID INCREASE SECTION】

(2) 【REMOVAL OF HEART RATE IN RAPID INCREASE SECTION】

START TIME OF RAPID INCREASE SECTION

END TIME OF RAPID INCREASE SECTION

# FIG.17

FIXED WINDOW WIDTH (210 MINUTES)

SLIDING

FIXED SHIFT WIDTH (5 MINUTES)

WINDOW DATA (NUMBER i)

WINDOW DATA (NUMBER i+1)

START TIME          END TIME

MEAL START CANDIDATE TIME

# FIG.18

CHEST ACCELERATION DURING MEAL (ACCELERATION IN FRONT-AND-BACK DIRECTION)

RECIPROCAL MOVEMENT PATTERN APPEARS IN FRONT-AND-BACK DIRECTION DURING MEAL

TIME

# FIG.19

MARGIN WIDTH (FIXED)

EXPLANATION VARIABLE 2

SUPPORT VECTORS

SET MARGIN TO EXTEND THROUGH SUPPORT VECTORS OF BOTH CLASSES

EXPLANATION VARIABLE 1

# FIG.20

**【YOUR LIFESTYLE HABIT FOR ONE WEEK】**

**【HEALTH SUPPORT ADVICES】**

·FORM HABIT OF TAKING BREAKFAST.
·HAVE DINNER EARLY. TAKING DINNER
FIVE HOURS BEFORE SLEEP IS BEST.
·GET EXERCISE.

# FIG.21

【CHANGE IN YOUR WEIGHT】

POSSIBILITY OF OBESITY

THERE IS POSSIBILITY OF OBESITY

STANDARD WEIGHT

15TH DAY 16TH DAY 17TH DAY 18TH DAY 19TH DAY 20TH DAY 21TH DAY

【ANALYSIS RESULT FOR YOU】

·CAUSES FOR INCREASE IN YOUR WEIGHT ARE AS FOLLOWS
(1)NUMBER OF TIMES OF MEALS IS LARGE
⇒HAVE BREAKFAST AND FORM REGULAR DIETARY HABIT
(2)MEAL IS TAKEN BEFORE SLEEP FREQUENTLY
⇒TAKING MEAL BEFORE SLEEP HAMPERS DIGESTION, AND CAUSES OBESITY. TAKE DINNER EARLY.

【YOUR LIFESTYLE HABIT】

9/21
9/20
9/19
9/18
9/17
9/16
9/15

(1)NUMBER OF TIMES OF MEALS IS LARGE

(2)MEAL IS TAKEN BEFORE SLEEP FREQUENTLY

:MEAL
:EXERCISE
:SLEEP

0:00    6:00    12:00    18:00    24:00

# FIG.22

| TIME | 09-02 10:59:30 | 09-02 11:00:00 | 09-02 11:00:30 | 09-02 11:01:30 | ... | 09-02 16:58:30 | 09-02 16:59:00 | 09-02 15:42:00 |
|---|---|---|---|---|---|---|---|---|
| HEART RATE | -- | 61.8 | 62.0 | 61.9 | ... | 64.5 | 64.2 | 64.2 |

# FIG.23

| TIME | 09-02 10:59:59. 900 | 09-02 11:00:00. 000 | 09-02 11:00:00. 200 | 09-02 11:00:00. 300 | ... | 09-02 16:59:59. 700 | 09-02 16:59:59. 800 | 09-02 16:59:59. 900 |
|---|---|---|---|---|---|---|---|---|
| ACCELERATI-ON (FRONT-AND-BACK DIRECTION) | 975 | 930 | 907 | 900 | ... | 856 | 877 | 877 |
| ACCELERATI-ON (LATERAL DIRECTION) | -221 | -223 | -227 | -229 | ... | -234 | -233 | -233 |
| ACCELERATI-ON (GRAVITY DIRECTION) | 108 | 110 | 112 | 167 | ... | 187 | 188 | 188 |

# FIG.24

| TIME | 09-02 11:00:00. 000 | 09-02 11:00:00. 200 | 09-02 11:00:00. 300 | ... | 09-02 16:59:59. 700 | 09-02 16:59:59. 800 | 09-02 16:59:59. 900 |
|---|---|---|---|---|---|---|---|
| ACCELERATI-ON (FRONT-AND-BACK DIRECTION) | 930 | 907 | 900 | ... | 856 | 877 | 877 |
| ACCELERATI-ON (LATERAL DIRECTION) | -223 | -227 | -229 | ... | -234 | -233 | -233 |
| ACCELERATI-ON (GRAVITY DIRECTION) | 510 | 512 | 567 | ... | 587 | 588 | 588 |

⇩

| SPECITY NUMBER OF TIMES OF EATING ACTIONS AND PRESENCE OF EXERCISE |
|---|

⇩

| TIME | 09-02 11:00:00 | ... | 09-02 12:41:30 | 09-02 12:42:00 | 09-02 12:42:30 | ... | 09-02 15:42:00 |
|---|---|---|---|---|---|---|---|
| HEART RATE | 61.8 | ... | 63.4 | 65.4 | 67.4 | ... | 64.2 |
| NUMBER OF TIMES OF EATING ACTIONS | 1 | ... | 0 | 3 | 2 | ... | 0 |
| EXERCISE PERIOD | 0(*) | ... | 0 | 0 | 0 | ... | 0 |

# FIG.25

EXERCISE START TIME    EXERCISE END TIME    EXERCISE END TIME + 5 MINUTES

| TIME | 09-02 11:00:00 | ... | 09-02 12:25:30 | 09-02 12:26:00 | ... | 09-02 12:33:00 | 09-02 12:33:30 | ... | 09-02 12:38:00 | ... | 09-02 15:42:00 |
|---|---|---|---|---|---|---|---|---|---|---|---|
| HEART RATE | 61.8 | ... | 62.4 | 65.4 | ... | 74.7 | 71.1 | ... | 67.4 | ... | 64.2 |
| NUMBER OF TIMES OF EATING ACTIONS | 0 | ... | 0 | 0 | ... | 0 | 0 | ... | 0 | ... | 0 |
| EXERCISE PERIOD | 0 | ... | 0 | 1 | ... | 1 | 0 | ... | 0 | ... | 0 |

REMOVE HEART RATES

| TIME | 09-02 11:00:00 | ... | 09-02 12:25:30 | 09-02 12:26:00 | ... | 09-02 12:33:00 | 09-02 12:33:30 | ... | 09-02 12:38:00 | ... | 09-02 15:42:00 |
|---|---|---|---|---|---|---|---|---|---|---|---|
| HEART RATE | 61.8 | ... | 62.4 | --- | ... | --- | --- | ... | --- | ... | 64.2 |
| NUMBER OF TIMES OF EATING ACTIONS | 0 | ... | 0 | 0 | ... | 0 | 0 | ... | 0 | ... | 0 |
| EXERCISE PERIOD | 0 | ... | 0 | 1 | ... | 1 | 0 | ... | 0 | ... | 0 |

EP 3 231 362 A1

# FIG.26

| WINDOW DATA NUMBER | WINDOW DATA START TIME | WINDOW DATA END TIME | T1 | T2 | ... | TN | MEAL START TIME |
|---|---|---|---|---|---|---|---|
| 1 | 09-02 11:00:00 | 09-02 14:30:00 | 61.8 | 62.0 | ... | 63.2 | 09-02 11:30:00 |
| 2 | 09-02 11:05:00 | 09-02 14:35:00 | 63.1 | 62.4 | ... | 64.1 | 09-02 11:35:00 |
| 3 | 09-02 11:10:00 | 09-02 14:40:00 | 62.5 | 59.2 | ... | 63.2 | 09-02 11:40:00 |
| ⋮ | ⋮ | ⋮ | ⋮ | ⋮ | ⋮ | ⋮ | ⋮ |
| M | 09-02 20:25:00 | 09-02 23:55:00 | 62.7 | 65.0 | ... | 58.0 | 09-02 20:55:00 |

SERIES OF HEART RATES FOR
FIXED WINDOW WIDTH

# FIG.27

SERIES OF HEART RATES

| WINDOW DATA NUMBER | WINDOW DATA START TIME | WINDOW DATA END TIME | 09-02 12:12:00 | ... | 09-02 15:42:00 | MEAL START TIME |
|---|---|---|---|---|---|---|
| i | 09-02 12:12:00 | 09-02 15:42:00 | 61.8 | ... | 63.2 | 09-02 12:42:00 |

⇩

CALCULATE FEATURE QUANTITIES

⇩

| WINDOW DATA NUMBER | WINDOW DATA START TIME | FIRST PEAK AREA | SECOND PEAK AREA | SECOND PEAK RECOVERY VELOCITY | SECOND PEAK INCREASE VELOCITY | FIRST PEAK AMPLITUDE | SECOND PEAK AMPLITUDE | PRE-MEAL HEART RATE | PRE-MEAL AREA |
|---|---|---|---|---|---|---|---|---|---|
| i | 09-02 12:42:00 | 17.3 | 16.2 | ? | ? | ? | ? | ? | ? |

EP 3 231 362 A1

# FIG.28

MEAL START TIME

PRE-MEAL PERIOD

| TIME | 09-02<br>12:12:00 | ... | 09-02<br>15:42:00 | ... | 09-02<br>15:42:00 |
|---|---|---|---|---|---|
| HEART<br>RATE | 61.8 | ... | 67.4 | ... | 63.2 |

CALCULATE LOWEST HEART RATE

| PRE-MEAL<br>HEART RATE |
|---|
| 59.0 |

# FIG.29

MEAL START TIME

| TIME | 09-02 12:12:00 | ... | 09-02 12:42:00 | ... | 09-02 12:56:30 | 09-02 12:57:00 | ... | 09-02 15:42:00 |
|---|---|---|---|---|---|---|---|---|
| HEART RATE | 61.8 | ... | 71.3 | ... | 79.4 | 75.3 | ... | 67.5 |
| RISE | 2.8 | ... | 15.3 | ... | 20.4 | 16.3 | ... | 8.5 |

PRE-MEAL PERIOD — MEAL PERIOD — POST-MEAL PERIOD

⇨ CALCULATE RISE AVERAGE ⇨

| WINDOW DATA NUMBER | MEAL START TIME | FIRST PEAK AREA | SECOND PEAK AREA | SECOND PEAK RECOVERY VELOCITY | SECOND PEAK INCREASE VELOCITY | FIRST PEAK AMPLITUDE | SECOND PEAK AMPLITUDE | PRE-MEAL HEART RATE | PRE-MEAL AREA |
|---|---|---|---|---|---|---|---|---|---|
| i | 09-02 12:42:00 | 17.3 | 16.2 | ? | ? | ? | ? | 59.0 | 3.1 |

# FIG.30

# FIG.31

MAXIMUM HEART RATE IN MEAL PERIOD

| TIME | 09-02 12:42:00 | ... | 09-02 12:49:00 | ... | 09-02 12:56:30 |
|---|---|---|---|---|---|
| HEART RATE | 71.3 | ... | 85.2 | ... | 75.3 |

| WINDOW DATA NUMBER | MEAL START CANDIDATE TIME | FIRST PEAK AREA | SECOND PEAK AREA | SECOND PEAK RECOVERY VELOCITY | SECOND PEAK INCREASE VELOCITY | FIRST PEAK AMPLITUDE | SECOND PEAK AMPLITUDE | PRE-MEAL HEART RATE | PRE-MEAL AREA |
|---|---|---|---|---|---|---|---|---|---|
| i | 09-02 12:42:00 | 17.3 | 16.2 | ? | ? | 85.2 | ? | 59.0 | 3.1 |

EP 3 231 362 A1

# FIG.32

MAXIMUM HEART RATE IN POST-MEAL PERIOD

| TIME | 09-02 12:57:00 | ... | 09-02 13:19:00 | ... | 09-02 15:42:00 |
|---|---|---|---|---|---|
| HEART RATE | 65.3 | ... | 83.7 | ... | 75.3 |

| WINDOW DATA NUMBER | MEAL START CANDIDATE TIME | FIRST PEAK AREA | SECOND PEAK AREA | SECOND PEAK RECOVERY VELOCITY | SECOND PEAK INCREASE VELOCITY | FIRST PEAK AMPLITUDE | SECOND PEAK AMPLITUDE | PRE-MEAL HEART RATE | PRE-MEAL AREA |
|---|---|---|---|---|---|---|---|---|---|
| i | 09-02 12:42:00 | 17.3 | 16.2 | ? | ? | 85.2 | 83.7 | 59.0 | 3.1 |

EP 3 231 362 A1

# FIG.33

EP 3 231 362 A1

START TIME OF POST-MEAL PERIOD

MAXIMUM HEART RATE TIME OF SECOND PEAK

| TIME | 09-02 12:57:00 | ... | 09-02 13:19:00 | ... | 09-02 15:42:30 |
|---|---|---|---|---|---|
| HEART RATE | 65.3 | ... | 83.7 | ... | 75.3 |

EXECUTE LINEAR REGRESSION

| | CALCULA-TION RESULT |
|---|---|
| INCLINATION PARAMETER (bpm/hour) | 17.1 |
| INTERCEPT PARAMETER (bpm) | 66.9 |

INCREASE RESPONSE VELOCITY

| WINDOW DATA NUMBER | MEAL START TIME | FIRST PEAK AREA | SECOND PEAK AREA | SECOND PEAK RECOVERY VELOCITY | SECOND PEAK INCREASE VELOCITY | FIRST PEAK AMPLITUDE | SECOND PEAK AMPLITUDE | PRE-MEAL HEART RATE | PRE-MEAL AREA |
|---|---|---|---|---|---|---|---|---|---|
| i | 12:42:00 | 17.3 | 16.2 | ? | 17.1 | 85.2 | 83.7 | 59.0 | 3.1 |

# FIG.34

HEART RATE

LINEAR FUNCTION:
$f(t) = \alpha t + \beta$

$y_i$

APPROXIMATION ERROR:
$\{y_i - (\alpha t_i + \beta)\}^2$

TIME t

$t_1$ $\quad$ $t_i$ $\quad$ $t_N$

# FIG.35

EP 3 231 362 A1

|  | SECOND PEAK MAXIMUM HEART RATE TIME |  | END TIME OF SECOND PEAK PERIOD |
|---|---|---|---|
| TIME | 09-02 12:57:00 | ... | 09-02 13:19:00 | ... | 09-02 15:42:00 |
| HEART RATE | 65.3 | ... | 83.7 | ... | 75.3 |

EXECUTE LINEAR REGRESSION

|  | CALCULA-TION RESULT |
|---|---|
| INCLINATION PARAMETER (bpm/hour) | -6.4 |
| INTERCEPT PARAMETER (bpm) | 83.9 |

RECOVERY RESPONSE VELOCITY

| WINDOW DATA NUMBER | MEAL START TIME | FIRST PEAK AREA | SECOND PEAK AREA | SECOND PEAK RECOVERY VELOCITY | SECOND PEAK INCREASE VELOCITY | FIRST PEAK AMPLITUDE | SECOND PEAK AMPLITUDE | PRE-MEAL HEART RATE | PRE-MEAL AREA |
|---|---|---|---|---|---|---|---|---|---|
| i | 12:42:00 | 17.3 | 16.2 | -6.4 | 17.1 | 85.2 | 83.7 | 59.0 | 3.1 |

# FIG.36

| WINDOW DATA NUMBER | MEAL START TIME | FIRST PEAK AREA | SECOND PEAK AREA | SECOND PEAK RECOVERY VELOCITY | SECOND PEAK INCREASE VELOCITY | FIRST PEAK AMPLITUDE | SECOND PEAK AMPLITUDE | PRE-MEAL HEART RATE | PRE-MEAL AREA |
|---|---|---|---|---|---|---|---|---|---|
| 1 | 11:30:00 | 5.0 | 4.2 | -2.4 | 6.1 | 70.2 | 83.7 | 59.0 | 3.1 |
| 2 | 11:35:00 | 4.5 | 3.2 | -0.2 | 4.3 | 67.2 | 83.7 | 64.2 | 4.1 |
| ⋮ | ⋮ | ⋮ | ⋮ | ⋮ | ⋮ | ⋮ | ⋮ | ⋮ | ⋮ |
| M | 20:55:00 | 17.3 | 16.2 | -6.4 | 7.3 | 76.9 | 88.1 | 56.1 | 8.1 |

EP 3 231 362 A1

# FIG.37

| TIME | NUMBER OF TIMES OF EATING ACTIONS |
|---|---|
| 09-02  11:00:00 | 0 |
| 09-02  11:00:30 | 0 |
| ⋮ | ⋮ |
| 09-02  20:55:00 | 0 |

# FIG.38

| TERMINAL NUMBER | MEAL START TIME | FIRST PEAK AREA | SECOND PEAK AREA | SECOND PEAK RECOVERY VELOCITY | SECOND PEAK INCREASE VELOCITY | FIRST PEAK AMPLITUDE | SECOND PEAK AMPLITUDE | PRE-MEAL HEART RATE | PRE-MEAL AREA | MEAL CLASS |
|---|---|---|---|---|---|---|---|---|---|---|
| XXX | 08-29 12:20:00 | 17.0 | 12.2 | -7.4 | 16.1 | 90.5 | 85.7 | 62.0 | 8.1 | ● |
| XXX | 08-30 12:35:00 | 16.5 | 22.2 | -12.2 | 18.3 | 69.2 | 83.7 | 64.2 | 2.1 | ● |
| ⋮ | ⋮ | ⋮ | ⋮ | ⋮ | ⋮ | ⋮ | ⋮ | ⋮ | ⋮ | ⋮ |
| XXX | 08-30 20:50:00 | 4.5 | 3.2 | -0.2 | 4.3 | 67.2 | 83.7 | 64.2 | 4.1 | × |

# FIG.39

EP 3 231 362 A1

| SUPPORT VECTOR NUMBER | WEIGHT | MEAL START TIME | FIRST PEAK AREA | SECOND PEAK AREA | SECOND PEAK RECOVERY VELOCITY | SECOND PEAK INCREASE VELOCITY | FIRST PEAK AMPLITUDE | SECOND PEAK AMPLITUDE | PRE-MEAL HEART RATE | PRE-MEAL AREA | MEAL CLASS |
|---|---|---|---|---|---|---|---|---|---|---|---|
| 1 | 0.42 | 08-29 12:20:00 | 17.0 | 12.2 | -7.4 | 16.1 | 90.5 | 85.7 | 62.0 | 8.1 | ●(I⁺) |
| ⋮ | ⋮ | ⋮ | ⋮ | ⋮ | ⋮ | ⋮ | ⋮ | ⋮ | ⋮ | ⋮ | ⋮ |
| L | 0.11 | 08-30 20:50:00 | 4.5 | 3.2 | -0.2 | 4.3 | 67.2 | 83.7 | 64.2 | 4.1 | ×(I⁻) |

## FIG.40

ESTIMATION RESULT OF
PRESENCE OF MEAL

| WINDOW DATA NUMBER | MEAL START CANDIDATE TIME | FIRST PEAK AREA | SECOND PEAK AREA | SECOND PEAK RECOVERY VELOCITY | SECOND PEAK INCREASE VELOCITY | FIRST PEAK AMPLITUDE | SECOND PEAK AMPLITUDE | PRE-MEAL AMPLITUDE | PRE-MEAL AREA | OUTPUT VALUE OF ESTIMATI-ON MODEL | DETERMI-NATION RESULT OF PRESENCE OF MEAL |
|---|---|---|---|---|---|---|---|---|---|---|---|
| 1 | 11:30:00 | 5.0 | 4.2 | -2.4 | 6.1 | 70.2 | 83.7 | 59.0 | 3.1 | -0.91 | × |
| 2 | 11:35:00 | 4.5 | 3.2 | -0.2 | 4.3 | 67.2 | 83.7 | 64.2 | 4.1 | -0.65 | × |
| ⋮ | ⋮ | ⋮ | ⋮ | ⋮ | ⋮ | ⋮ | ⋮ | ⋮ | ⋮ | ⋮ | ⋮ |
| m | 12:42:00 | 17.3 | 16.2 | -6.4 | 17.1 | 85.2 | 83.7 | 59.0 | 3.1 | 0.87 | ● |
| ⋮ | ⋮ | ⋮ | ⋮ | ⋮ | ⋮ | ⋮ | ⋮ | ⋮ | ⋮ | ⋮ | ⋮ |
| M | 20:55:00 | 17.3 | 16.2 | -6.4 | 7.3 | 76.9 | 88.1 | 56.1 | 8.1 | -0.99 | × |

OBSERVATION FEATURE QUANTITY LIST

# FIG.41

| WINDOW DATA NUMBER | MEAL START CANDI- DATE TIME | DETERMINA- TION RESULT OF PRESENCE OF MEAL |
|---|---|---|
| 1 | 11:30:00 | × |
| 2 | 11:35:00 | × |
| ⋮ | ⋮ | ⋮ |
| m | 12:42:00 | ● |
| ⋮ | ⋮ | ⋮ |
| M | 20:55:00 | × |

| TIME | NUMBER OF TIMES OF EATING ACTIONS |
|---|---|
| 09-02  11:00:00 | 0 |
| 09-02  11:00:30 | 0 |
| ⋮ | ⋮ |
| 09-02  12:42:00 | 2 |
| ⋮ | ⋮ |
| 09-02  13:02:00 | 0 |
| ⋮ | ⋮ |
| 09-02  20:55:00 | 0 |

TWENTY MINUTES

| TOTAL NUMBER OF TIMES OF EATING ACTIONS | FINAL DETERMINATION RESULT (THRESHOLD LEVEL=10) |
|---|---|
| 17 | ● |

EP 3 231 362 A1

# FIG.42

```
                        ( START )
                            │
                            ▼
┌─────────────────────────────────────────────┐
│ ACQUIRE HEART RATE DATA AND ACCELERATION     │~ S101
│ DATA                                          │
└─────────────────────────────────────────────┘
                            │
                            ▼
┌─────────────────────────────────────────────┐
│ DETERMINE EXERCISE PERIOD FROM               │~ S102
│ ACCELERATION DATA                             │
└─────────────────────────────────────────────┘
                            │
                            ▼
┌─────────────────────────────────────────────┐
│ DETERMINE EATING ACTIONS FROM ACCELERATION   │~ S103
│ DATA                                          │
└─────────────────────────────────────────────┘
                            │
                            ▼
┌─────────────────────────────────────────────┐
│ REMOVE NOISE HEART RATE                       │~ S104
└─────────────────────────────────────────────┘
                            │
                            ▼
┌─────────────────────────────────────────────┐
│ PREPARE PIECES OF WINDOW DATA WITH           │~ S105
│ SEGMENTED HEART RATE DATA                     │
└─────────────────────────────────────────────┘
                            │
                            ▼
┌─────────────────────────────────────────────┐
│ SELECT ONE PIECE OF WINDOW DATA              │~ S106
└─────────────────────────────────────────────┘
                            │
                            ▼
┌─────────────────────────────────────────────┐
│ FEATURE QUANTITY CALCULATION PROCESSING      │~ S107
└─────────────────────────────────────────────┘
                            │
                            ▼
                     S108
        NO      ╱ HAVE ALL PIECES OF ╲
       ◄────────  WINDOW DATA BEEN
                ╲    SELECTED?       ╱
                            │ YES
                            ▼
┌─────────────────────────────────────────────┐
│ TRANSMIT DATA FROM SENSOR TERMINAL TO        │~ S109
│ SERVER APPARATUS                              │
└─────────────────────────────────────────────┘
                            │
                            ▼
┌─────────────────────────────────────────────┐
│ PREPARE MEAL ESTIMATION MODEL                │~ S110
└─────────────────────────────────────────────┘
                            │
                            ▼
┌─────────────────────────────────────────────┐
│ DETERMINE PRESENCE OF MEAL USING MEAL        │~ S111
│ ESTIMATION MODEL                              │
└─────────────────────────────────────────────┘
                            │
                            ▼
                     S112
        ╱ DOES ANY WINDOW DATA ╲      NO
        ╲ CLASSIFIED INTO       ╱ ───────►
          ╲ "MEAL" EXIST?     ╱
                            │ YES
                            ▼
                     S113
        ╱ DO AT LEAST            ╲     NO
        ╲ PREDETERMINED NUMBER OF ╱ ──►
          ╲ TIMES OF EATING ACTIONS
            ╲ EXIST?            ╱
                            │ YES
                            ▼
┌─────────────────────────────────────────────┐
│ SPECIFY MEAL TIME                            │~ S114
└─────────────────────────────────────────────┘
                            │
                            ▼
┌─────────────────────────────────────────────┐
│ PROVIDE SERVICES                             │~ S115
└─────────────────────────────────────────────┘
                            │
                            ▼
                        ( END )
```

# FIG.43

```
        ┌─────────────┐
        │    START    │
        └──────┬──────┘
               ▼
┌───────────────────────────┐
│  CALCULATE BASELINE OF     │ ── S201
│  HEART RATE               │
└──────────┬────────────────┘
           ▼
┌───────────────────────────┐
│  CALCULATE AREA OF FIRST   │ ── S202
│  PEAK REGION              │
└──────────┬────────────────┘
           ▼
┌───────────────────────────┐
│  CALCULATE AREA OF SECOND  │ ── S203
│  PEAK REGION              │
└──────────┬────────────────┘
           ▼
        ┌─────────────┐
        │     END     │
        └─────────────┘
```

# FIG.44

START

SPECIFY MEASUREMENT TIME OF MAXIMUM HEART RATE OF FIRST PEAK — S301

CALCULATE INCREASE VELOCITY OF FIRST PEAK BY FUNCTION APPROXIMATION — S302

CALCULATE RECOVERY VELOCITY FROM FIRST PEAK BY FUNCTION APPROXIMATION — S303

SPECIFY MEASUREMENT TIME OF MAXIMUM HEART RATE OF SECOND PEAK — S304

CALCULATE INCREASE VELOCITY OF SECOND PEAK BY FUNCTION APPROXIMATION — S305

CALCULATE RECOVERY VELOCITY FROM SECOND PEAK BY FUNCTION APPROXIMATION — S306

END

# FIG.45

```
        ┌─────────────┐
        │    START    │
        └─────────────┘
               │
               ▼
┌───────────────────────────────┐
│   DERIVE MAXIMUM HEART RATE    │ ⟋S401
│        OF FIRST PEAK           │
└───────────────────────────────┘
               │
               ▼
┌───────────────────────────────┐
│   DERIVE MAXIMUM HEART RATE    │ ⟋S402
│        OF SECOND PEAK          │
└───────────────────────────────┘
               │
               ▼
        ┌─────────────┐
        │     END     │
        └─────────────┘
```

# FIG.46

```
                    ┌──────────────┐
                    │    START     │
                    └──────┬───────┘
                           ▼
        ┌───────────────────────────────────┐
        │ SPECIFY MEASUREMENT TIME OF        │
        │ MAXIMUM HEART RATE OF FIRST        │──S501
        │ PEAK                               │
        └──────────────────┬────────────────┘
                           ▼
        ┌───────────────────────────────────┐
        │ CALCULATE INCREASE TIME OF         │──S502
        │ FIRST PEAK                         │
        └──────────────────┬────────────────┘
                           ▼
        ┌───────────────────────────────────┐
        │ CALCULATE RECOVERY VELOCITY        │
        │ FROM FIRST PEAK BY FUNCTION        │──S503
        │ APPROXIMATION                      │
        └──────────────────┬────────────────┘
                           ▼
        ┌───────────────────────────────────┐
        │ CALCULATE RECOVERY TIME FROM       │
        │ FIRST PEAK USING RECOVERY          │──S504
        │ VELOCITY FROM FIRST PEAK           │
        └──────────────────┬────────────────┘
                           ▼
        ┌───────────────────────────────────┐
        │ SPECIFY MEASUREMENT TIME OF        │
        │ MAXIMUM HEART RATE OF SECOND       │──S505
        │ PEAK                               │
        └──────────────────┬────────────────┘
                           ▼
        ┌───────────────────────────────────┐
        │ CALCULATE INCREASE TIME OF         │──S506
        │ SECOND PEAK                        │
        └──────────────────┬────────────────┘
                           ▼
        ┌───────────────────────────────────┐
        │ CALCULATE RECOVERY VELOCITY        │
        │ FROM SECOND PEAK BY FUNCTION       │──S507
        │ APPROXIMATION                      │
        └──────────────────┬────────────────┘
                           ▼
        ┌───────────────────────────────────┐
        │ CALCULATE RECOVERY TIME FROM       │
        │ SECOND PEAK USING RECOVERY         │──S508
        │ VELOCITY FROM SECOND PEAK          │
        └──────────────────┬────────────────┘
                           ▼
                    ┌──────────────┐
                    │     END      │
                    └──────────────┘
```

# FIG.47

```
         ┌─────────────┐
         │    START    │
         └─────────────┘
                │
                ▼
┌──────────────────────────────┐
│  DERIVE PRE-MEAL HEART RATE   │~S601
└──────────────────────────────┘
                │
                ▼
┌──────────────────────────────┐
│     DERIVE PRE-MEAL AREA      │~S602
└──────────────────────────────┘
                │
                ▼
         ┌─────────────┐
         │     END     │
         └─────────────┘
```

# FIG.48

EP 3 231 362 A1

<div align="center">

**INTERNATIONAL SEARCH REPORT**

</div>

| | International application No. |
|---|---|
| | PCT/JP2014/083059 |

| A. CLASSIFICATION OF SUBJECT MATTER |
|---|
| *A61B5/0245*(2006.01)i |

According to International Patent Classification (IPC) or to both national classification and IPC

| B. FIELDS SEARCHED |
|---|
| Minimum documentation searched (classification system followed by classification symbols) |
| A61B5/00-5/03 |

Documentation searched other than minimum documentation to the extent that such documents are included in the fields searched

| Jitsuyo Shinan Koho | 1922–1996 | Jitsuyo Shinan Toroku Koho | 1996–2015 |
|---|---|---|---|
| Kokai Jitsuyo Shinan Koho | 1971–2015 | Toroku Jitsuyo Shinan Koho | 1994–2015 |

Electronic data base consulted during the international search (name of data base and, where practicable, search terms used)
JSTPlus/JMEDPlus/JST7580(JDreamIII)

| C. DOCUMENTS CONSIDERED TO BE RELEVANT | | |
|---|---|---|
| Category* | Citation of document, with indication, where appropriate, of the relevant passages | Relevant to claim No. |
| X<br>Y<br>A | JP 2003-173375 A  (Toshiba Corp.),<br>20 June 2003 (20.06.2003),<br>paragraphs [0035] to [0038]; fig. 4<br>(Family: none) | 1,9,13<br>6<br>2-5,7-8,<br>10-12 |
| Y | JP 2011-115508 A  (Sharp Corp.),<br>16 June 2011 (16.06.2011),<br>claim 1<br>(Family: none) | 6 |
| A | JP 10-504739 A  (LANIADO, Eran, Meia),<br>12 May 1998 (12.05.1998),<br>entire text; fig. 2, 3<br>& US 5398688 A          & EP 771173 A1<br>& WO 1996/003076 A1      & AU 1918795 A<br>& IL 112382 A            & CA 2195547 A1 | 1-13 |

| ☒ | Further documents are listed in the continuation of Box C. | ☐ | See patent family annex. |
|---|---|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 06 March 2015 (06.03.15) | 17 March 2015 (17.03.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (July 2009)

**INTERNATIONAL SEARCH REPORT**

International application No.

PCT/JP2014/083059

C (Continuation). DOCUMENTS CONSIDERED TO BE RELEVANT

| Category* | Citation of document, with indication, where appropriate, of the relevant passages | Relevant to claim No. |
|---|---|---|
| A | JP 2010-158267 A (Sony Corp.), 22 July 2010 (22.07.2010), paragraph [0048] & US 2010/0174153 A1 & CN 101770547 A | 1-13 |
| A | JP 2007-48180 A (Yoriaki YAMAI), 22 February 2007 (22.02.2007), claim 1 (Family: none) | 1-13 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011115508 A
- JP 2012061790 A
- JP 2004081471 A
- JP 2010158267 A
- JP 2007048180 A
- JP 10504739 A
- JP 2003173375 A